# EUROPEAN PATENT SPECIFICATION

(11) **EP 4 288 134 B1**
(45) Date of publication and mention of the grant of the patent: **14.10.2026**
(21) Application number: 22749337.6
(22) Date of filing: 04.02.2022
(51) Int. Cl.: A61M 16/00, A61B 5/08, A61M 16/04, A61M 16/16, A61M 16/10, A61M 16/06, A61M 16/20

(54) **DETERMINING INSPIRATORY AND EXPIRATORY PARAMETERS IN RESPIRATORY FLOW THERAPY SYSTEMS**
BESTIMMUNG VON INSPIRATORISCHEN UND EXSPIRATORISCHEN PARAMETERN IN ATEMFLUSSTHERAPIESYSTEMEN
DÉTERMINATION DE PARAMÈTRES INSPIRATOIRES ET EXPIRATOIRES DANS DES SYSTÈMES DE THÉRAPIE PAR FLUX RESPIRATOIRE

(30) Priority: 05.02.2021 US 202163146184 P
(43) Date of publication of application: 13.12.2023
(73) Proprietor: Fisher & Paykel Healthcare Limited, 2013 Auckland (NZ)
(72) Inventor: GULLEY, Anton Kim, 2013 Auckland (NZ)
(74) Representative: Forresters IP LLP
(86) International application number: PCT/IB2022/050967
(87) International publication number: WO 2022/167982

(56) References cited:
- WO-A1-2011/089491
- WO-A1-2015/136405
- US-A1- 2006 070 624
- US-A1- 2006 249 148
- US-A1- 2006 278 223
- US-A1- 2012 037 159
- US-A1- 2020 197 642
- US-A1- 2020 360 636
- US-A1- 2020 360 636

## Description

### FIELD OF THE DISCLOSURE

The present disclosure relates to methods and systems for providing a respiratory flow therapy to a patient. In particular, the present disclosure relates to determining one or more inspiratory and/or expiratory parameters during use of an unsealed respiratory apparatus (i.e. open respiratory apparatus) by a patient.

### BACKGROUND

Breathing assistance apparatuses are used in various environments such as hospital, medical facility, residential care, or home environments to deliver a flow of gases to users or patients. A breathing assistance or respiratory therapy apparatus (collectively, "respiratory apparatus" or "respiratory devices") may be used to deliver supplementary oxygen or other gases with a flow of gases, and/or a humidification apparatus to deliver heated and humidified gases. A respiratory apparatus may allow adjustment and control over characteristics of the gases flow, including flow rate, temperature, gases concentration, humidity, pressure, etc. Sensors, such as flow sensors and/or pressure sensors are used to measure characteristics of the gases flow.

US patent application publication 2012/0037159 discloses a controller or processor for implementing detection of respiratory related conditions, such asynchrony, associated with the use of a respiratory treatment apparatus or ventilator.

US patent application publication 2006/0278223 discloses a system for automatic ongoing adjustment of the cycling-off time of ventilator inflation phase during assisted ventilation in accordance with true respiratory rate of a patient.

US patent application publication 2006/0249148 discloses a method and apparatus for non-invasively determining the time onset and end of patient respiratory efforts.

US patent application publication 2020/0360636 discloses a system for providing continuous air pressure therapy, including a display for presenting one or more questions to a user and determining settings based on answers received.

### SUMMARY

Respiratory devices can monitor and determine various parameters related to a patient's use of the device. The parameter data can inform clinicians about a patient's health, use of the respiratory devices and/or progress in the patient's respiratory functions. The data can also be used to improve the functionality of the respiratory device itself.

Inspiration and expiration by a patient using a respiratory device can affect the gases flow in the device. This is because when the patient inhales through a patient interface, such as a mask or nasal cannula, the resistance to the gases flow in the patient interface decreases; when the patient exhales, the resistance to the gases flow in the patient interface increases. Some parameters, such as respiratory rate, are determined by monitoring variations due to the inspiration and expiration in a flow parameter signal.

In a sealed system, this inhalation and exhalation is relatively easy to measure. Sealed systems use substantially sealed masks e.g. a full face mask or a nasal mask and are used to provide therapies like CPAP (Continuous positive airway pressure) therapy or BiLevel pressure therapy. However, in an unsealed system, such as a nasal high flow system, patient inhalation and exhalation is more difficult to determine because of the open nature of the system.

The present disclosure provides methods and processes for performing analysis of a gases flow parameter to determine one or more breathing parameter estimates or ratios relating to inspiration time, expiration time, total respiration time, for a patient using a respiratory apparatus delivering nasal high flow therapy (e.g. a nasal high flow apparatus) via an unsealed patient interface (e.g. a nasal cannula). The use of an unsealed interface makes the respiratory apparatus an "open" respiratory apparatus, meaning there is a significant leak at the patient interface due to the unsealed nature of the patient interface. In one configuration, the breathing parameter estimates and/or representative parameters or ratios are derived from a sensed or representative flow rate signal or flow rate data determined during operation of the respiratory apparatus.

The determination of the breathing parameter estimates, parameters and/or ratios may be fed to other control functions of the respiratory apparatus and/or associated patient monitoring devices, displayed to the patient or clinician numerically, graphically or otherwise, used to trigger notifications and/or alarms, and/or used for feedback and/or analytics to determine efficacy of the treatment, such as short and long-term trend information of the patient's breathing parameters during treatment or respiratory functions, or to prompt the clinician or user to adjust therapy parameters or settings to improve treatment outcomes.

The processes disclosed herein can be used when the patient interface is a non-sealed device, such as a nasal cannula in a nasal high flow therapy, or via an unsealed tracheal interface to provide tracheal high flow therapy. Nasal high flow and tracheal high flow are collectively known as "high flow therapy".

The present invention relates to a respiratory apparatus according to claim 1 that is configured to provide a flow of gases to a user for respiratory therapy comprising: a flow generator that is operable to generate a flow of gases; a controller that is operatively connected to the flow generator and operable to control a flow rate of the flow of gases by controlling the flow generator, wherein the controller is configured during operation to: receive flow rate data indicative of or representing the flow rate of the flow of gases; process the flow rate data to extract or generate breathing data indicative of or representing the patient's breathing or respiration from the flow rate data; and process the breathing data to calculate one or more breathing parameter ratios representative of the ratio between inspiration time and/or expiration time to total respiration time for the patient's breathing cycle.

In a configuration, the controller may be configured extract or generate the breathing data from the flow rate data by at least removing unwanted components from the flow rate data that are attributable to or caused by the flow generator.

In a configuration, the flow generator may comprise a motor that drives an impeller to generate the flow of gases, and the controller is configured to extract or generate the breathing data from the flow rate data by removing unwanted components from the flow rate data that are attributable or caused by the motor of the flow generator.

In a configuration, the controller may be further configured to determine whether the quality of the flow rate data is suitable for processing to extract or generate breathing data and such that breathing data is only extracted from flow rate data that is determined to be suitable for processing.

In a configuration, the controller may be further configured to process the data of the parameter of the flow of gases to remove noise. In a configuration, the controller is configured to remove noise relating to the effect of a motor on the parameter of the flow of gases. In a configuration, the controller is configured to receive data regarding a motor speed, and the parameter of the flow of gases is discarded if the motor speed is below a pre-set threshold. In a configuration, the controller is configured to discard the parameter of the flow of gases if the controller determines the parameter of the flow of gases is of insufficient quality. In a configuration, the parameter of the flow of gases is of insufficient quality because it includes large transient peaks.

In a configuration, the controller may be configured to process the breathing data to generate the one or more breathing parameters ratios by: fitting a function or line to a selected portion of the breathing data, and calculating the one or more breathing parameter ratios based at least partly on one or more parameters defining the fitted function or line.

The controller is configured to process the breathing data to generate the one or more breathing parameters ratios by: fitting a function or line to a selected portion of the breathing data, calculating a breathing parameter value based at least partly on one or more parameters defining the fitted function or line, and determining one or more of the breathing parameter ratios based on a rolling average of the breathing parameter value.

In a configuration, the breathing parameter value may be a Boolean or Categorical value or data type that is calculated or determined based on one or more parameters defining the fitted function or line.

In a configuration, the controller may be configured to process the breathing data to generate the one or more breathing parameters ratios by: calculating a breathing parameter value based on the breathing parameter data, and determining one or more of the breathing parameter ratios based on a rolling average of the breathing parameter value.

In a configuration, the breathing parameter value may be a Boolean or Categorical value or data type that is calculated or determined based on one or more parameters defining the fitted function or line.

In a configuration, the controller may be further configured to apply one or more noise correction and/or noise cleaning and/or noise filtering processes during the process of determining the one or more breathing parameter ratios. **In** a configuration, the controller may apply noise correction and/or noise cleaning and/or noise filtering based on a signal to noise function or variable.

In a configuration, the controller may be further configured to determine and generate a signal quality indicator that represents or is indicative of the quality of the determined one or more breathing parameter ratios. **In** one example configuration, the signal quality indicator may be determined based on a variance value associated with or calculated based on the breathing parameter ratio. **In** one example configuration, the signal quality indicator may be determined based on a standard deviation value associated with or calculated based on the breathing parameter ratio. In one example configuration, the signal quality indicator may be determined based on comparing the determined one or more breathing parameter ratios to predetermined upper and/or lower thresholds.

In a configuration, the controller may be further configured to: receive or calculate respiratory rate data representing or indicative of the patient's respiratory rate, and calculate one or more additional breathing parameter ratios and/or breathing parameters based on the initially calculated primary breathing parameter ratio(s) and the respiratory rate data.

In a configuration, the one or more additional breathing parameter ratios calculated may comprise any additional ratio between any combination or permutation of two of the following breathing parameters: inspiration time, expiration time, and total respiration time.

In a configuration, the one or more additional breathing parameters calculated may comprise any one or more of the following: inspiration time, expiration time, and/or total respiration time.

In a configuration, the respiratory rate data may be received or retrieved from a device or sensor operatively connected to the respiratory apparatus.

In a configuration, the controller may be configured to calculate the respiratory rate data from the breathing data extracted from the flow rate data.

In a configuration, the controller may be configured to calculate the respiratory rate data based at least partly on analysing or determining the dominant frequency component in the breathing data.

In a configuration, the controller may be configured to implement one or more functions based on one or more of the calculated breathing parameter ratio(s) and/or breathing parameters.

In a configuration, the controller may be configured to display one or more of the calculated breathing parameter ratios and/or breathing parameters on a display of the apparatus.

In a configuration, the controller may be configured to display one or more of the calculated breathing parameter ratios and/or breathing parameters numerically and/or as a graph, plot or chart.

In a configuration, the controller may be configured to trigger alarms and/or notifications for display on the respiratory apparatus based on analysing the calculated breathing parameter ratios and/or breathing parameters.

In a configuration, the controller may be configured to apply trend analysis to one or more of the calculated breathing parameter ratios and/or breathing parameters, and triggers alarms and/or notifications for display based on the trend analysis and configurable trend thresholds.

In a configuration, the controller may be configured to modify or alter operational and/or therapy settings based on the calculated breathing parameter ratios and/or breathing parameters.

The respiratory apparatus is configured or operable to deliver high flow therapy to a patient via an unsealed interface.

In a configuration, the respiratory apparatus may further comprise a humidifier that is configured to heat and/or humidify the flow of gases, and wherein the flow generator and humidifier rare integrated within or provided in a common main housing.

In a configuration, the flow rate data may be received from one or more flow rate sensors in the main housing.

In a configuration, the flow rate data may be received from one or more flow rate sensors that are configured to sense the flow rate of the flow of gases in a flow path of the main housing.

In another aspect the present disclosure relates to a method of controlling a respiratory apparatus that is configured to provide a flow of gases to a user for respiratory therapy, the apparatus comprising: a flow generator that is operable to generate a flow of gases; a controller that is operatively connected to the flow generator and operable to control a flow rate of the flow of gases by controlling a the flow generator, wherein the method is executable or implemented by the controller and comprises: receiving flow rate data indicative of or representing the flow rate of the flow of gases; processing the flow rate data to extract or generate breathing data indicative of or representing the patient's breathing or respiration from the flow rate data; and processing the breathing data to calculate one or more breathing parameter ratios representative of the ratio between inspiration time and/or expiration time to total respiration time for the patient's breathing cycle.

In another aspect the present disclosure relates to a respiratory apparatus that is configured to provide a flow of gases to a user for respiratory therapy comprising: a flow generator that is operable to generate a flow of gases; a controller that is operatively connected to the flow generator and operable to control a flow rate of the flow of gases by controlling the flow generator, wherein the controller is configured during operation to: receive flow rate data indicative of or representing the flow rate of the flow of gases; process the flow rate data to extract or generate breathing data indicative of or representing the patient's breathing or respiration from the flow rate data; receive respiratory rate data or calculate respiratory rate data indicative of the patient's respiratory rate based at least partly on the breathing data extracted from the flow rate data; and process the breathing data and respiratory rate data to calculate one or more breathing parameters indicative of inspiration time and/or expiration time of the patient's breathing cycle.

In another aspect the present disclosure relates to a method of controlling a respiratory apparatus that is configured to provide a flow of gases to a user for respiratory therapy, the apparatus comprising: a flow generator that is operable to generate a flow of gases; a controller that is operatively connected to the flow generator and operable to control a flow rate of the flow of gases by controlling a the flow generator, wherein the method is executable or implemented by the controller and comprises: receiving flow rate data indicative of or representing the flow rate of the flow of gases; processing the flow rate data to extract or generate breathing data indicative of or representing the patient's breathing or respiration from the flow rate data; receiving respiratory rate data or calculating respiratory rate data indicative of the patient's respiratory rate based at least partly on the breathing data extracted from the flow rate data; and processing the breathing data and respiratory rate data to calculate one or more breathing parameters indicative of inspiration time and/or expiration time of the patient's breathing cycle.

In another aspect the present disclosure relates to a respiratory system configured to deliver a respiratory therapy to a patient, the system also configured to provide information related to the patient's breathing, the system comprising: a respiratory device comprising a controller, wherein the controller is configured to: receive data of a first parameter of a flow of gases or representative of performance of a component of the device, the first parameter indicative of the patient's respiration, determine, based on the data of the first parameter, one or more breathing parameters representing inspiration time and/or expiration time, and/or breathing parameter ratios representative of any ratio between any combination or permutation of two of the following breathing parameters: inspiration time, expiration time, and total respiration time, for the patient's breathing cycle.

In a configuration, the data of the first parameter may comprise an absolute value of the first parameter.

In a configuration, the data of the first parameter may comprise a variation of the first parameter.

In a configuration, the variation may be determined by subtracting a target value of the first parameter from the measured value of the first parameter.

In a configuration, the variation may be determined by subtracting an estimated effect of a second parameter from the measured value of the first parameter.

In one example the first parameter may be flow rate.

In one example the second parameter may be motor speed.

In one example the system is a non-sealed system that utilises an unsealed patient interface. An unsealed patient interface is an interface that includes a large leak i.e. air (or gases) exhaled by the patient freely leaks around and/or through the interface.

In a configuration, the system may comprise a patient interface, wherein the patient interface comprises a nasal cannula or a tracheostomy interface. The nasal cannula and tracheostomy interface are unsealed interfaces.

In a configuration, the system may be configured to deliver a nasal high flow therapy.

In a configuration, the system may comprise a humidifier configured to humidify the gases flow to a patient.

In a configuration, the system may comprise a display configured to receive from one or more processors of the controller and display information related to the determined breathing parameters and/or breathing parameter ratios.

In a configuration, the controller may be configured to: generate flow parameter variation data based on the data of the first parameter; select a portion of the flow parameter variation data; and generate, based at least partly on the selected portion of the flow parameter variation data, the one or more breathing parameters and/or ratios.

In a configuration, the controller may be further configured to fit or apply one or more functions to the selected portion of the flow parameter variation data, and to generate the one or more breathing parameters and/or ratios based at least partly on one or more parameters defining the one or more fitted function(s).

In a configuration, the controller may be configured to perform a least squares fit to fit the one or more functions to the selected portion of the flow parameter variation data.

In a configuration, the curve generated by the one or more functions may be a straight line.

In a configuration, the curve generated by the one or more functions may be a horizontal line.

In a configuration, the one or more functions may be algebraic.

In a configuration, the one or more functions may be transcendental.

In a configuration, the one or more functions may generate a line of best fit.

In a configuration, the first parameter may be indicative of or is flow rate.

In a configuration, the flow rate may be the total flow rate.

In a configuration, the flow parameter variation data may be generated by subtracting a target value of the first parameter from a measured value of the first parameter.

In a configuration, the controller may be further configured to receive data of a second parameter of the flow of gases or representative of performance of a second component of the device, and wherein the flow parameter variation data is generated by subtracting an estimated effect of the second parameter from a measured value of the first parameter.

In a configuration, the second parameter may be indicative of or is motor speed.

In a configuration, the flow parameter variation data may be generated by subtracting a first average value of the first parameter from a second average value of the first parameter.

In a configuration, the second average value may be based on measured values of the first parameter.

In a configuration, the first average value of the first parameter may be determined by applying an ongoing filter to the first parameter.

In a configuration, the portion of the flow parameter variation data may comprise data relating to a time period within a predefined time period.

In a configuration, the portion of the flow parameter variation data may represent a length of time.

In a configuration, the length of time may be such that signal noise is filtered out of the breathing parameters and/or ratios.

In another aspect the present disclosure relates a respiratory apparatus that is configured to provide a flow of gases to a user for respiratory therapy comprising: a housing; a flow generator positioned within the housing that is operable to generate a flow of gases; a display; and a controller that is operatively connected to the flow generator and operable to control a flow rate of the flow of gases by controlling the flow generator, the controller operatively connected to the display and operable to control display of information on the display, wherein the controller is configured during operation to: receive flow rate data indicative of or representing the flow rate of the flow of gases; and process the flow rate data to determine one or more breathing parameter ratios representative of the ratio between inspiration time and/or expiration time to total respiration time for the patient's breathing cycle.

In a configuration, the controller may be configured to transmit the one or more breathing parameter ratios to the display, and the display is configured to display the one or more breathing parameter ratios on the display.

In one example the breathing parameter ratios may be related to inspiratory and/or expiratory parameters.

In a configuration, the controller may be configured to determine a trend of the one or more breathing parameter ratios, the controller configured to transmit to the display the trend of the one or more breathing parameters and the display configured to display the trend of the one or more breathing parameter ratios.

In a configuration, the trend may be indicative of the change of the one or more breathing parameter ratios over a time period.

In a configuration, the respiratory apparatus may be a high flow therapy apparatus. The high flow therapy apparatus may be configured to deliver high flow therapy. The apparatus may comprise a conduit and an unsealed interface, the conduit fluidly coupling the flow generator to the unsealed interface. Optionally the high flow therapy apparatus comprises a humidifier.

In a configuration, the controller may be configured to determine or receive respiratory rate data, and the controller may be further configured to determine additional breathing parameters based on the respiratory rate data.

In another aspect the present disclosure relates to a high flow therapy apparatus, the high flow therapy apparatus comprises: a flow generator operable to generate a flow of gases; a humidifier in fluid communication with the flow generator; a flow sensor; a display; and a controller, the controller is operatively coupled to the flow generator, the humidifier, the flow sensor and the display, the controller configured to control operation of the flow generator, humidifier and display, the controller further configured to: receive flow rate data indicative of or representing the flow rate of the flow of gases from the flow sensor, the controller configured to determine one or more breathing parameter ratios and/or breathing parameters, and the controller configured to perform one or more actions and/or functions based on the determined breathing parameter ratios and/or breathing parameters.

In a configuration, the breathing parameter ratio may be representative of the ratio between inspiration time and/or expiration time to total respiration time for the patient's breathing cycle.

In a configuration, the one or more actions and/or functions may comprise any one or more of: transmitting the one or more breathing parameter ratios and/or breathing parameters to the display, such that the ratios and/or parameters are displayed on the display; controlling the flow generator based on the one or more breathing parameter ratios and/or breathing parameters; and/or controlling the humidifier based on the one or more breathing parameter ratios and/or breathing parameters.

In another aspect, the present disclosure relates to an electronically-implemented method defined by software code or coded instructions that are executable or implemented by a computer, processor, or controller to carry out any one or more of the methods or aspects described above.

In another aspect, the present disclosure relates to a non-transitory computer-readable medium having stored thereon computer executable instructions that, when executed on a processing device or devices, cause the processing device or devices to perform any one or more of the methods or aspects described above.

In another aspect, the present disclosure relates to a respiratory apparatus that is configured to provide a flow of gases to a user for respiratory therapy comprising: a housing; an inlet in the housing; an outlet in the housing; a flow generator positioned within the housing; a humidifier in fluid communication with the flow generator; a gases path extending from the inlet though the flow generator and the humidifier to the outlet, the gases path allowing gases to flow from the inlet to the outlet; a flow sensor positioned in the gases path and configured to measure flow rate data indicative or representative of the flow rate of the flow of gases; a user interface positioned on a face of the housing or embedded in the housing, the user interface comprising a display and/or one or more input elements that are used to input data; and a controller that is operatively connected to the flow generator and operable to control a flow rate of the flow of gases by controlling the flow generator, wherein the controller is configured during operation to: receive flow rate data indicative of or representing the flow rate of the flow of gases from the flow sensor; process the flow rate data to determine breathing data indicative of or representing the patient's breathing or respiration from the flow rate data, wherein the data indicative of or representing patient's breathing is determined by filtering the flow rate data using an appropriate function on a selected portion of the flow rate data to provide filtered flow rate data; and process the breathing data to calculate one or more breathing parameter ratios representative of the ratio between inspiration time and/or expiration time to total respiration time for the patient's breathing cycle, process the flow rate data to determine a respiratory rate of a user based on determining a dominant frequency in the filtered flow rate data, or, receive an input of the user's respiratory rate via the user interface, determine one or more of an inspiration time, expiration time, total respiration time, inspiration time to expiration time ratio or expiration time to inspiration time ratio or an expiratory time to total respiration time ratio or an inspiration time to total respiration time ratio, and transmit one of the determined parameters to the user interface and display one or more of the determined parameters on the display.

In one configuration the respiratory apparatus is a high flow apparatus that is configured to provide nasal high flow therapy via an unsealed interface. The respiratory apparatus may be configured for use in a hospital setting or may be configured for an out of hospital setting e.g. homecare or hospice etc.

In one configuration the controller is configured to check the one or more determined parameters with a threshold parameter, and the controller further configured to control the flow generator to adjust the delivered flow rate and/or control an amount of supplementary gases received into the gases path via the inlet based on a difference between the determined parameter and the threshold. The threshold may be manually input by a clinician or other authorized party.

### BRIEF DESCRIPTION OF THE DRAWINGS

These and other features, aspects, and advantages of the present disclosure are described with reference to the drawings of certain embodiments, which are intended to schematically illustrate certain embodiments and not to limit the disclosure.
**Figure 1** shows schematically a respiratory system configured to provide a respiratory therapy to a patient.
**Figure 2** is a front view of an example respiratory device with a humidification chamber in position and a raised handle/lever.
**Figure 3** is a top view corresponding to Figure 2.
**Figure 4** is a right side view corresponding to Figure 2.
**Figure 5** is a left side view corresponding to Figure 2.
**Figure 6** is a rear view corresponding to Figure 2.
**Figure 7** is a front left perspective view corresponding to Figure 2.
**Figure 8** is a front right perspective view corresponding to Figure 2.
**Figure 9** is a bottom view corresponding to Figure 2.
**Figure 10** shows an example configuration of an air and oxygen inlet arrangement of a respiratory device.
**Figure 11** shows another example configuration of an air and oxygen inlet arrangement of the respiratory device.
**Figure 12** is a transverse sectional view showing further detail of the air and oxygen inlet arrangement of Figure 11.
**Figure 13** is another transverse sectional view showing further detail of the air and oxygen inlet arrangement of Figure 11.
**Figure 14** is a longitudinal sectional view showing further detail of the air and oxygen inlet arrangement of Figure 11.
**Figure 15** is an exploded view of upper and lower chassis components of a main housing of the respiratory device.
**Figure 16** is a front left side perspective view of the lower chassis of the main housing showing a housing for receipt of a motor/sensor module sub-assembly.
**Figure 17** is a first underside perspective view of the main housing of the respiratory device showing a recess inside the housing for the motor/sensor module sub-assembly.
**Figure 18** is a second underside perspective view of the main housing of the respiratory device showing the recess for the motor/sensor module sub-assembly.
**Figure 19A** illustrates a block diagram of a control system interacting with and/or providing control and direction to components of a respiratory system.
**Figure 19B** illustrates a block diagram of an example controller.
**Figure 20** illustrates a block diagram of a motor and sensor module.
**Figure 21** illustrates a sensing chamber of an example motor and sensor module.
**Figure 22** shows a summary flow diagram of an embodiment of a breathing parameter determination process in a first configuration.
**Figure 23** shows a detailed flow diagram of an embodiment of a breathing parameter determination process in a second configuration.

### DETAILED DESCRIPTION

### 1. Overview of Example Respiratory Apparatus

The methods and processes of determining the breathing parameter estimates and/or ratios relating to or indicative of inspiration time, expiration time, and/or total respiration time, will be described in the context of an example respiratory apparatus 10 that is configured or operable to provide nasal high flow therapy via a unsealed patient interface. This is intended as a non-limiting example. It will be appreciated that the methods and processes may be applied to other respiratory apparatus and/or to other modes of operation and/or modes of therapy delivered by such apparatus.

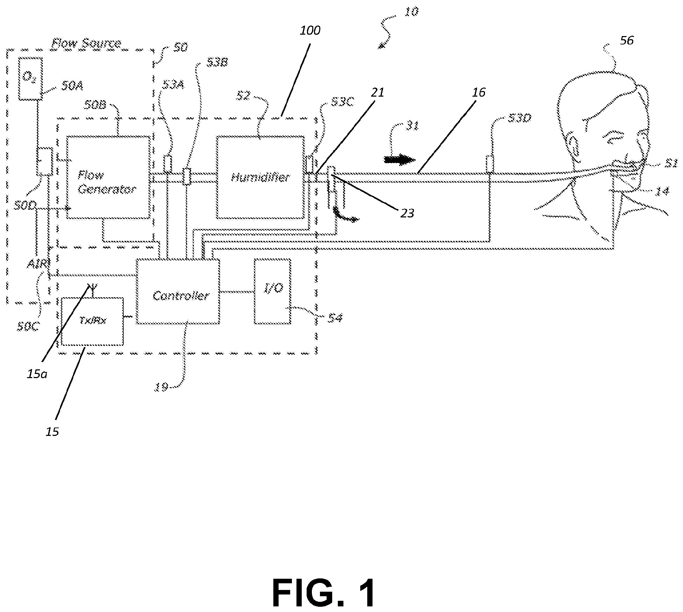

A schematic representation of the example respiratory apparatus 10 is provided in Figure 1.

The respiratory apparatus 10 (or 'respiratory system') comprises a flow source 50 for providing a high flow gas 31 such as air, oxygen, air blended with oxygen, or a mix of air and/or oxygen and one or more other gases. Alternatively, the breathing assistance apparatus can have a connection for coupling to a flow source. As such, the flow source might be considered to form part of the apparatus or be separate to it, depending on context, or even part of the flow source forms part of the apparatus, and part of the flow source falls outside of the apparatus. In short, depending on the configuration (some components may be optional), the system can include a combination of components selected from the following:
- a flow source
- humidifier for humidifying the gas-flow,
- conduit (e.g. dry line or heated breathing tube),
- patient interface,
- non-return valve
- filter

The apparatus or system will be described in more detail.

The flow source could be an in-wall supply of oxygen, a tank of oxygen 50A, a tank of other gas and/or a high flow apparatus with a flow generator 50B. Figure 1 shows a flow source 50 with a flow generator 50B, with an optional air inlet 50C and optional connection to an O2 source (such as tank or O2 generator) 50A via a shut off valve and/or regulator and/or other gas flow control 50D, but this is just one option. The flow generator 50B can control flows delivered to the patient 56 using one or more valve, or optionally the flow generator 50B can comprise a blower. The flow source could be one or a combination of a flow generator 50B, O2 source 50A, air source 50C as described. The flow source 50 is shown as part of the apparatus 10, although in the case of an external oxygen tank or in-wall source, it may be considered a separate component, in which case the apparatus has a connection port to connect to such flow source. The flow source provides a (preferably high) flow of gas that can be delivered to a patient via a delivery conduit 16, and patient interface 51.

The patient interface 51 may be an unsealed (non-sealing) interface (for example when used in high flow therapy) such as a non-sealing nasal cannula, or a sealed (sealing) interface (for example when used in CPAP) such as a nasal mask, full face mask, or nasal pillows. In some embodiments, the patient interface 51 is a non-sealing patient interface which would for example help to prevent barotrauma (e.g. tissue damage to the lungs or other organs of the respiratory system due to difference in pressure relative to the atmosphere). In some embodiments, the patient interface 51 is a sealing mask that seals with the patient's nose and/or mouth. The patient interface may be a nasal cannula with a manifold and nasal prongs, and/or a face mask, and/or a nasal pillows mask, and/or a nasal mask, and/or a tracheostomy interface, or any other suitable type of patient interface. The flow source could provide a base gas flow rate of between, e.g. 0.5 litres/min and 375 litres/min, or any range within that range, or even ranges with higher or lower limits. Details of the ranges and nature of flow rates will be described later.

A humidifier 52 can optionally be provided between the flow source 50 and the patient to provide humidification of the delivered gas. One or more sensors 53A, 53B, 53C, 53D such as flow, oxygen fraction, pressure, humidity, temperature or other sensors can be placed throughout the system and/or at, on or near the patient 56. Alternatively, or additionally, sensors from which such parameters can be derived could be used. In addition, or alternatively, the sensors 53A-53D can be one or more physiological sensors for sensing patient physiological parameters such as, heart rate, oxygen saturation, partial pressure of oxygen in the blood, respiratory rate, partial pressure of CO2 in the blood. Alternatively, or additionally, sensors from which such parameters can be derived could be used. Other patient sensors could comprise EEG sensors, torso bands to detect breathing, and any other suitable sensors. In some configurations the humidifier may be optional, or it may be preferred due to the advantages of humidified gases helping to maintain the condition of the airways. One or more of the sensors might form part of the apparatus, or be external thereto, with the apparatus having inputs for any external sensors. The sensors can be coupled to or send their output to a controller 19.

In some configurations, the respiratory system 10 can include a sensor 14 for measuring the oxygen fraction of air the patient inspires. In some examples, the sensor 14 can be placed on the patient interface 51, to measure or otherwise determine the fraction of oxygen proximate (at/near/close to) the patient's mouth and/or nose. In some configurations, the output from the sensor 14 is sent to a controller 19 to assist control of the respiratory system 10 alter operation accordingly. The controller 19 is coupled to the flow source 50, humidifier 52 and sensor 14. In some configurations, the controller 19 controls these and other aspects of the respiratory system 10 as described herein. In some examples, the controller can operate the flow source 50 to provide the delivered flow of gas at a desired flow rate high enough to meet or exceed a user's (i.e. patient's) inspiratory demand. The flow rate is provided is sufficient that ambient gases are not entrained as the user (i.e. patient) inspires. In some configurations, the sensor 14 can convey measurements of oxygen fraction at the patient mouth and/or nose to a user, who can input the information to the respiratory system 10/controller 19.

An optional non-return valve 23 may be provided in the breathing conduit 16. A filter or filters may be provided at the air inlet 50C and/or inlets to the flow generator 50B to filter the incoming gases before they are pressurized into a high flow gas 31 by to the flow generator 50B.

The breathing assistance apparatus 10 could be an integrated or a separate componentbased arrangement, generally shown in the dotted box 100 in Figure 1. In some configurations, the apparatus or system could be a modular arrangement of components. Furthermore, the apparatus or system may just comprise some of the components shown, not necessarily all are essential. Also, the conduit and patient interface do not have to be part of the system, and could be considered separate. Hereinafter it will be referred to as a breathing assistance apparatus or respiratory system, but this should not be considered limiting. Breathing assistance apparatus and respiratory system will be broadly considered herein to comprise anything that provides a flow rate of gas to a patient. Some such apparatus and systems include a detection system that can be used to determine if the flow rate of gas meets inspiratory demand.

The respiratory apparatus 10 can include a main device housing 100. The main device housing 100 can contain the flow generator 50B that can be in the form of a motor/impeller arrangement, an optional humidifier or humidification chamber 52, a controller 19, and an input/output I/O user interface 54. The user interface 54 can include a display and input device(s) such as button(s), a touch screen (e.g. an LCD screen), a combination of a touch screen and button(s), or the like. The controller 19 can include one or more hardware and/or software processors and can be configured or programmed to control the components of the system, including but not limited to operating the flow generator 50B to create a flow of gases for delivery to a patient, operating the humidifier or humidification chamber 52 (if present) to humidify and/or heat the gases flow, receiving user input from the user interface 54 for reconfiguration and/or user-defined operation of the respiratory apparatus 10, and outputting information (for example on the display) to the user. The user can be a patient, healthcare professional, or others.

With continued reference to Figure 1, a patient breathing conduit 16 can be coupled to a gases flow outlet (gases outlet or patient outlet port) 21 in the main device housing 100 of the respiratory apparatus 10, and be coupled to a patient interface 17, such as a non-sealing interface like a nasal cannula with a manifold and nasal prongs. The patient breathing conduit 16 can also be a tracheostomy interface, or other unsealed interfaces.

The gases flow can be generated by the flow generator 50B, and may be humidified, before being delivered to the patient via the patient breathing conduit 16 through the patient interface 51. The controller 19 can control the flow generator 50B to generate a gases flow of a desired flow rate, and/or one or more valves to control mixing of air and oxygen or other breathable gas. The controller 19 can control a heating element in or associated with the humidification chamber 52, if present, to heat the gases to a desired temperature that achieves a desired level of temperature and/or humidity for delivery to the patient. The patient breathing conduit 16 can have a heating element, such as a heater wire, to heat gases flow passing through to the patient. The heating element can also be under the control of the controller 19.

The humidifier 52 of the apparatus is configured to combine or introduce humidity with or into the gases flow. Various humidifier 52 configurations may be employed. In one configuration, the humidifier 52 can comprise a humidification chamber that is removable. For example, the humidification chamber may be partially or entirely removed or disconnected from the flow path and/or apparatus. By way of example, the humidification chamber may be removed for refilling, cleaning, replacement and/or repair for example. In one configuration, the humidification chamber may be received and retained by or within a humidification compartment or bay of the apparatus, or may otherwise couple onto or within the housing of the apparatus.

The humidification chamber of the humidifier 52 may comprise a gases inlet and a gases outlet to enable connection into the gases flow path of the apparatus. For example, the flow of gases from the flow generator 50B is received into the humidification chamber via its gases inlet and exits the chamber via its gases outlet, after being heated and/or humidified.

The humidification chamber contains a volume of liquid, typically water or similar. In operation, the liquid in the humidification chamber is controllably heated by one or more heaters or heating elements associated with the chamber to generate water vapour or steam to increase the humidity of the gases flowing through the chamber.

In one configuration, the humidifier is a Passover humidifier. In another configuration, the humidifier may be a non-Passover humidifier.

In one configuration, the humidifier may comprise a heater plate, for example associated or within a humidification bay that the chamber sits on for heating. The chamber may be provided with a heat transfer surface, e.g a metal insert, plate or similar, in the base or other surface of the chamber that interfaces or engages with the heater plate of the humidifier.

In another configuration, the humidification chamber may comprise an internal heater or heater elements inside or within the chamber. The internal heater or heater elements may be integrally mounted or provided inside the chamber, or may be removable from the chamber.

The humidification chamber may be any suitable shape and/or size. The location, number, size, and/or shape of the gases inlet and gases outlet of the chamber may be varied as required. In one configuration, the humidification chamber may have a base surface, one or more side walls extending up from the base surface, and an upper or top surface. In one configuration, the gases inlet and gases outlet may be position on the same side of the chamber. In another configuration, the gases inlet and gases outlet may be on different surfaces of the chamber, such as on opposite sides or locations, or other different locations.

In some configurations, the gases inlet and gases outlet may have parallel flow axes. In some configurations, the gases inlet and gases outlet may be positioned at the same height on the chamber.

The apparatus 10 can use ultrasonic transducer(s), flow sensor(s) such as a thermistor flow sensor, pressure sensor(s), temperature sensor(s), humidity sensor(s), or other sensors, in communication with the controller 19, to monitor characteristics of the gases flow and/or operate the system 10 in a manner that provides suitable therapy. The gases flow characteristics can include gases concentration, flow rate, pressure, temperature, humidity, or others. The sensors 53A, 53B, 53C, 53D, 14, such as pressure, temperature, humidity, and/or flow sensors, can be placed in various locations in the main device housing 100, the patient conduit 16, and/or the patient interface 51. The controller 19 can receive output from the sensors to assist it in operating the respiratory apparatus 10 in a manner that provides suitable therapy, such as to determine a suitable target temperature, flow rate, and/or pressure of the gases flow. Providing suitable therapy can include meeting or exceeding a patient's inspiratory demand. In the illustrated embodiment sensors 53A, 53B, and 53C are positioned in the housing of the apparatus, sensor 53D in the patient conduit 16, and sensor 14 in the patient interface 51.

The apparatus 10 can include one or more communication modules to enable data communication or connection with one or more external devices or servers over a data or communication link or data network, whether wired, wireless or a combination thereof. In one configuration for example, the apparatus 10 can include a wireless data transmitter and/or receiver, or a transceiver 15 to enable the controller 19 to receive data signals in a wireless manner from the operation sensors and/or to control the various components of the system 10. The transceiver 15 or data transmitter and/or receiver module may have an antenna 15a as shown. In one example, the transceiver may comprise a Wi-Fi modem. Additionally, or alternatively, the data transmitter and/or receiver 15 can deliver data to a remote patient management system (i.e. remote server) or enable remote control of the system 10. The system 10 can include a wired connection, for example, using cables or wires, to enable the controller 19 to receive data signals from the operation sensors and/or to control the various components of the apparatus 10. The apparatus 10 may comprise one or more wireless communication modules. For example, the apparatus may comprise a cellular communication module such as for example a 3G, 4G or 5G module. The module 15 may be or may comprise a modem that enables the apparatus to communicate with a remote patient management system (not illustrated in the figures) using an appropriate communication network. The remote management system may comprise a single server or multiple servers or multiple computing devices implemented in a cloud computing network. The communication may be two-way communication between the apparatus and a patient management system (e.g. a server) or other remote system. The apparatus 10 may also comprise other wireless communication modules such as for example a Bluetooth module and/or a Wi-Fi module. The Bluetooth and/or WiFi module allow the apparatus to wirelessly send information to another device such as for example a smartphone or tablet or operate over a LAN (local area network) or Wireless LAN (WLAN). The apparatus may additionally, or alternatively, comprise a Near Field Communication (NFC) module to allow for data transfer and/or data communication.

For example, measured patient breathing parameter data (e.g., inspiratory, expiratory, and/or total respiratory time ratios) may be communicated to a remote patient management system (i.e. a remote server). The remote patient management system may be a single server or a network of servers or a cloud computing system or other suitable architecture for operating a remote patient management system. The remote patient management system (i.e. remote server) further includes memory for storing received data and various software applications or services that are executed to perform multiple functions. Then, for example, the remote patient management system (i.e. remote server) may communicate information or instructions to the system 10 at least in part dependent on the data received. For example, the nature of the data received may trigger the remote server (or a software application running on the remote server) to communicate an alert, alarm, or notification to the system 10. The remote patient management system may further store the received data for access by an authorized party such as a clinician or the patient or another authorized party. The remote patient management system may further be configured to generate reports in response to a request from an authorized party, and the breathing parameter data e.g. inspiratory, expiratory and/or total respiratory time ratios may be included into the generated reports. The reports may further comprise other patient breathing parameters e.g. respiratory rate or SpO2 and/or device parameters e.g. flow rate, humidity level.

The respiratory apparatus 10 may comprise a high flow therapy apparatus. High flow therapy as discussed herein is intended to be given its typical ordinary meaning, as understood by a person of skill in the art, which generally refers to a respiratory system delivering a targeted flow of humidified respiratory gases via an intentionally unsealed patient interface with flow rates generally intended to meet or exceed inspiratory flow of a user. Typical patient interfaces include, but are not limited to, a nasal or tracheal patient interface. Typical flow rates for adults often range from, but are not limited to, about fifteen litres per minute to about sixty litres per minute or greater. Typical flow rates for pediatric users (such as neonates, infants and children) often range from, but are not limited to, about one litre per minute per kilogram of user weight to about three litres per minute per kilogram of user weight or greater.

High flow therapy can also optionally include gas mixture compositions including supplemental oxygen and/or administration of therapeutic medicaments.

High flow therapy is often referred to as nasal high flow (NHF), humidified high flow nasal cannula (HHFNC), high flow nasal oxygen (HFNO), high flow therapy (HFT), or tracheal high flow (THF), among other common names. For example, in some configurations, for an adult patient 'high flow therapy' may refer to the delivery of gases to a patient at a flow rate of greater than or equal to about 10 litres per minute (10 LPM), such as between about 10 LPM and about 100 LPM, or between about 15 LPM and about 95 LPM, or between about 20 LPM and about 90 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow therapy apparatus with an adult patient, a neonatal, infant, or child patient, may deliver gases to the patient at a flow rate of between about 1 LPM and about 100 LPM, or at a flow rate in any of the sub-ranges outlined above.

High flow therapy can be effective in meeting or exceeding the patient's inspiratory demand, increasing oxygenation of the patient and/or reducing the work of breathing. Additionally, high flow therapy may generate a flushing effect in the nasopharynx such that the anatomical dead space of the upper airways is flushed by the high incoming gases flow. The flushing effect can create a reservoir of fresh gas available of each and every breath, while minimizing re-breathing of carbon dioxide, nitrogen, etc. High flow therapy can also increase expiratory time of the patient due to pressure during expiration. This in turn reduces the respiratory rate of the patient.

The patient interface for use in a high flow therapy can be a non-sealing interface to prevent barotrauma, which can include tissue damage to the lungs or other organs of the patient's respiratory system due to difference in pressure relative to the atmosphere. The patient interface can be a nasal cannula with a manifold and nasal prongs, and/or an unsealed tracheostomy interface, or any other suitable type of patient interface.

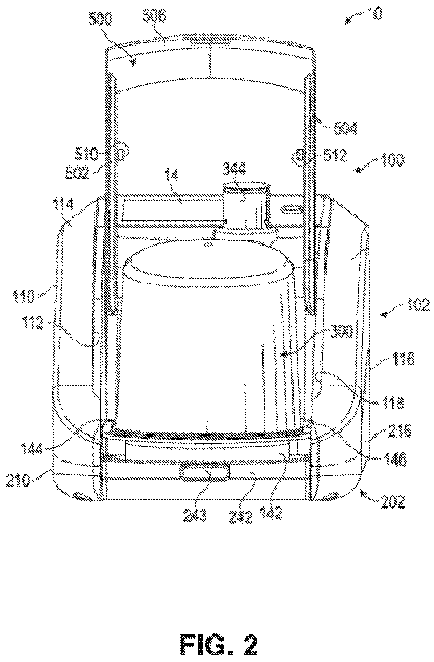

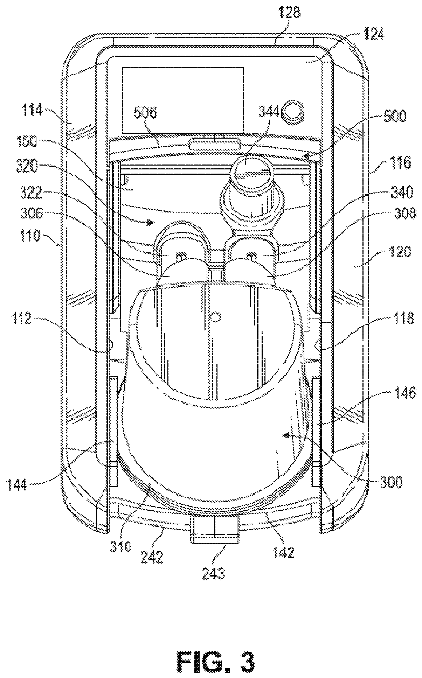

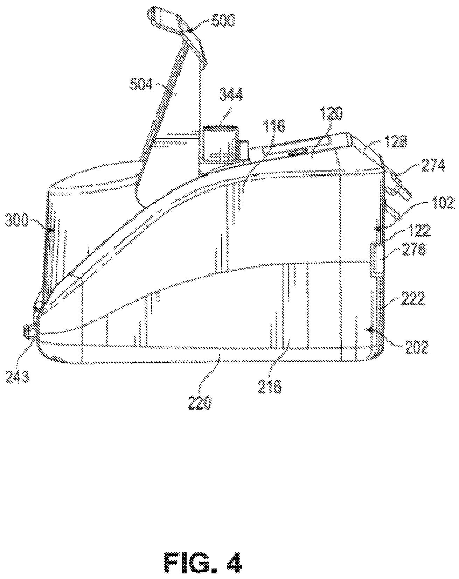

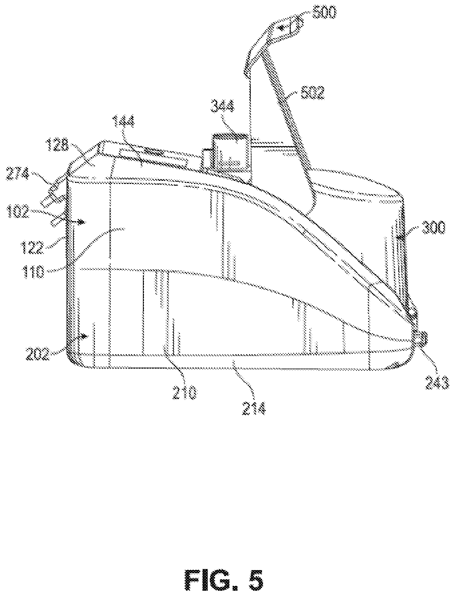

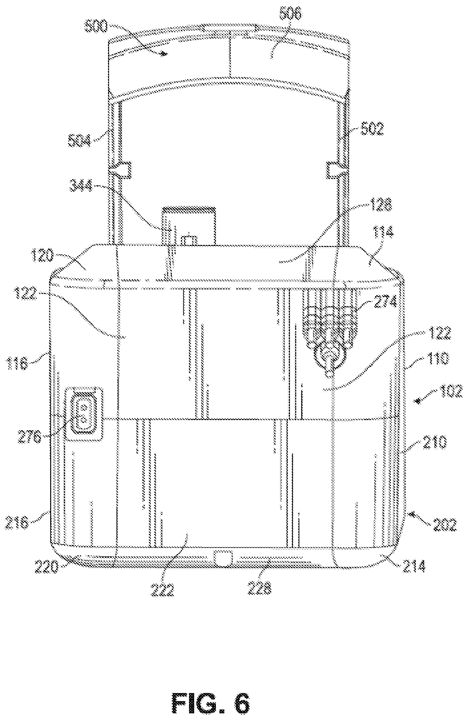

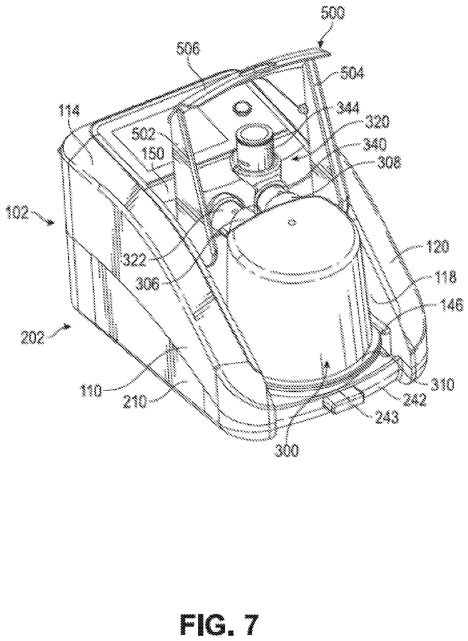

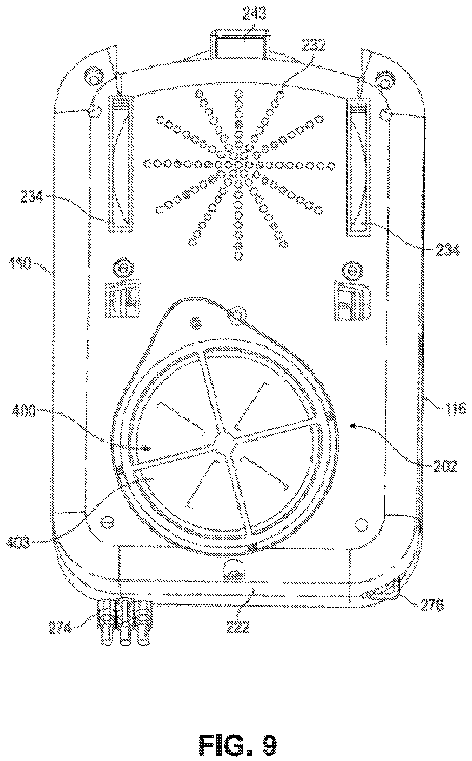

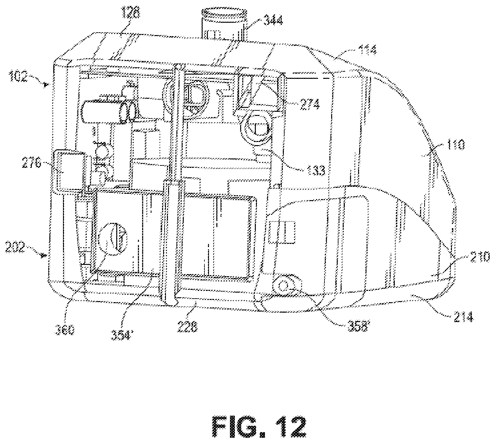

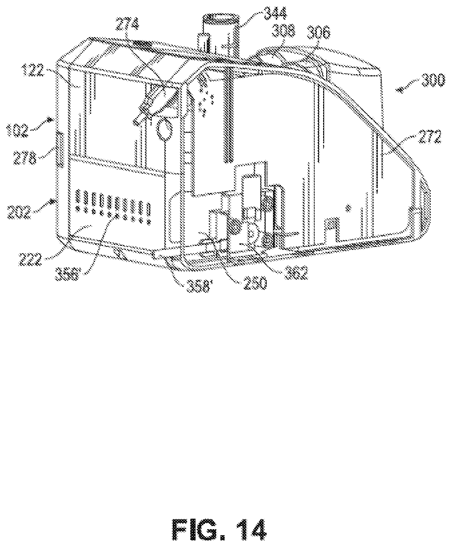

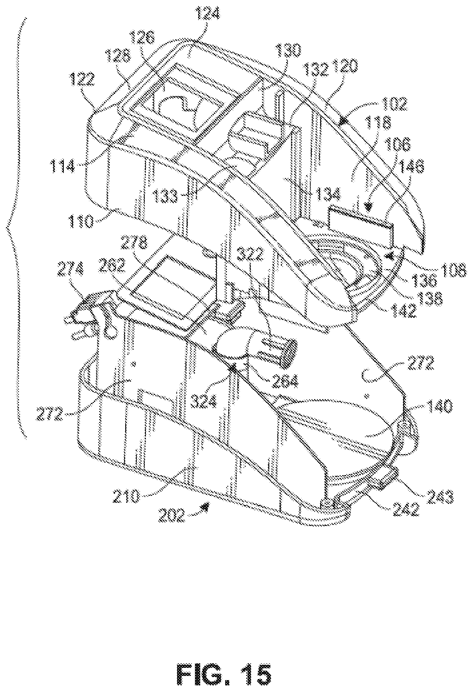

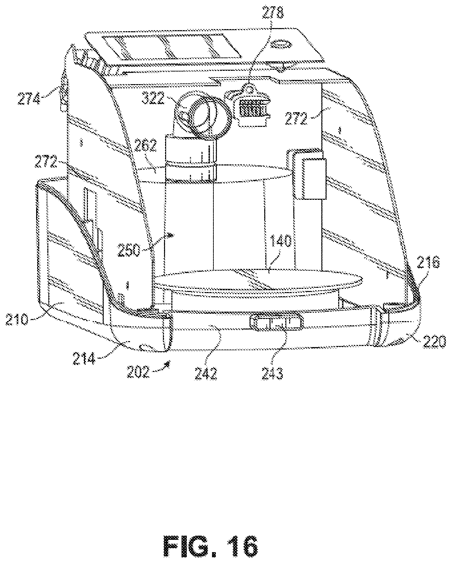

Figures 2 to 18 show an example respiratory device of the respiratory apparatus 10 having a main housing 100. The main housing 100 has a main housing upper chassis 102 and a main housing lower chassis 202. The main housing upper chassis 102 has a peripheral wall arrangement 106 (see Figure 15). The peripheral wall arrangement defines a humidifier or humidification chamber bay 108 for receipt of a removable humidification chamber 300. The removable humidification chamber 300 contains a suitable liquid such as water for humidifying gases that can be delivered to a patient.

In the form shown, the peripheral wall arrangement 106 of the main housing upper chassis 102 can include a substantially vertical left side outer wall 110 that is oriented in a front-to-rear direction of the main housing 100, a substantially vertical left side inner wall 112 that is oriented in a front-to-rear direction of the main housing 100, and an interconnecting wall 114 that extends between and interconnects the upper ends of the left side inner and outer walls 110, 112. The main housing upper chassis 102 can further include a substantially vertical right side outer wall 116 that is oriented in a front-to-rear direction of the main housing 100, a substantially vertical right side inner wall 118 that is oriented in a front-to-rear direction of the main housing 100, and an interconnecting wall 120 that extends between and interconnects the upper ends of the right side inner and outer walls 116, 118. The interconnecting walls 114, 120 are angled towards respective outer edges of the main housing 100, but can alternatively be substantially horizontal or inwardly angled.

The main housing upper chassis 102 can further include a substantially vertical rear outer wall 122. An upper part of the main housing upper chassis 102 can include a forwardly angled surface 124. The surface 124 can have a recess 126 for receipt of a display and user interface module 54. The display can be configured to display characteristics of sensed gas(es) in real time. The system can display the patient detection status of the patient interface. If the patient is not detected, the controller may not output or can stop outputting the respiratory rate value(s) and/or other parameters for display. The controller can also optionally output a message for display that no patient is detected at block 2708. An example of the message can be a "--" icon. An interconnecting wall 128 can extend between and interconnect the upper end of the rear outer wall 122 and the rear edge of the surface 124.

A substantially vertical wall portion 130 can extend downwardly from a front end of the surface 124. A substantially horizontal wall portion 132 can extend forwardly from a lower end of the wall portion 130 to form a ledge. A substantially vertical wall portion 134 can extend downwardly from a front end of the wall portion 132 and terminate at a substantially horizontal floor portion 136 of the humidification chamber bay 108. The left side inner wall 112, right side inner wall 118, wall portion 134, and floor portion 136 together can define the humidification chamber bay 108. The floor portion 136 of the humidification chamber bay 108 can have a recess 138 to receive a heater arrangement such as a heater plate 140 or other suitable heating element(s) for heating liquid in the humidification chamber 300 for use during a humidification process.

The main housing lower chassis 202 can be attachable to the upper chassis 102, either by suitable fasteners or integrated attachment features such as clips for example. The main housing lower chassis 202 can include a substantially vertical left side outer wall 210 that is oriented in a front-to-rear direction of the main housing 100 and is contiguous with the left side outer wall 110 of the upper chassis 102, and a substantially vertical right side outer wall 216 that is oriented in a front-to-rear direction of the main housing 100 and is contiguous with the right side outer wall 116 of the upper chassis 102. The main housing lower chassis 202 can further include a substantially vertical rear outer wall 222 that is contiguous with the rear outer wall 122 of the upper chassis 102.

The lower housing chassis 202 can have a lip 242 that is contiguous with the lip 142 of the upper housing chassis 102, and also forms part of the recess for receiving the handle portion 506 of the lever 500. The lower lip 242 can include a forwardly directed protrusion 243 that acts as a retainer for the handle portion 506 of the lever 500. Instead of the lever 500, the system can have a spring-loaded guard to retain the humidification chamber 300 in the humidification chamber bay 108.

An underside of the lower housing chassis 202 can include a bottom wall 230. Respective interconnecting walls 214, 220, 228 can extend between and interconnect the substantially vertical walls 210, 216, 222 and the bottom wall 230. The bottom wall 230 can include a grill 232 comprising a plurality of apertures to enable drainage of liquid in case of leakage from the humidification chamber 300 (e.g. from spills). The bottom wall 230 additionally can include elongated forward-rearward oriented slots 234. The slots 234 can additionally enable drainage of liquid in case of leakage from the humidification chamber 300, without the liquid entering the electronics housing. In the illustrated configuration, the slots 234 can be wide and elongate relative to the apertures of the grill 232 to maximize the drainage of liquid.

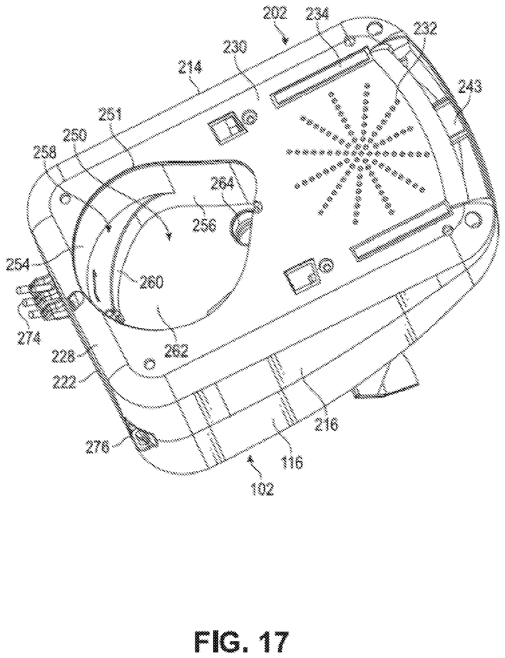

As shown in Figure 17 to 18, the lower chassis 202 can have a motor recess 250 for receipt of a motor and sensor module. The motor and sensor module may be non-removable from the main housing 100. The motor and sensor module can be removable from the main housing 100, as illustrated in Figures 17-18. A recess opening 251 can be provided in the bottom wall 230 adjacent a rear edge thereof, for receipt of a motor/sensor module. A continuous, gas impermeable, unbroken peripheral wall 252 can be integrally formed with the bottom wall 230 of the lower chassis 202 and extend upwardly from the periphery of the opening 251. A rearward portion 254 of the peripheral wall 252 has a first height, and a forward portion 256 of the peripheral wall 252 has a second height that is greater than the first height. The rearward portion 254 of the peripheral wall 252 terminates at a substantially horizontal step 258, which in turn terminates at an upper auxiliary rearward portion 260 of the peripheral wall 252. The forward portion 256 and upper auxiliary rearward portion 260 of the peripheral wall 252 terminate at a ceiling 262. All of the walls and the ceiling 262 can be continuous, gas impermeable, and unbroken other than the gases flow passage. Therefore, the entire motor recess 250 can be gas impermeable and unbroken, other than the gases flow passage.

The motor and sensor module can be insertable into the recess 250 and attachable to the lower chassis 202. Upon insertion of the motor and sensor module into the lower chassis 202, the gases flow passage tube 264 can extend through the downward extension tube 133 and be sealed by the soft seal.

The humidification chamber 300 can be fluidly coupled to the apparatus 10 in a linear slide-on motion in a rearward direction of the humidification chamber 300 into the chamber bay 108, from a position at the front of the housing 100 in a direction toward the rear of the housing 100. A gases outlet port 322 can be in fluid communication with the motor.

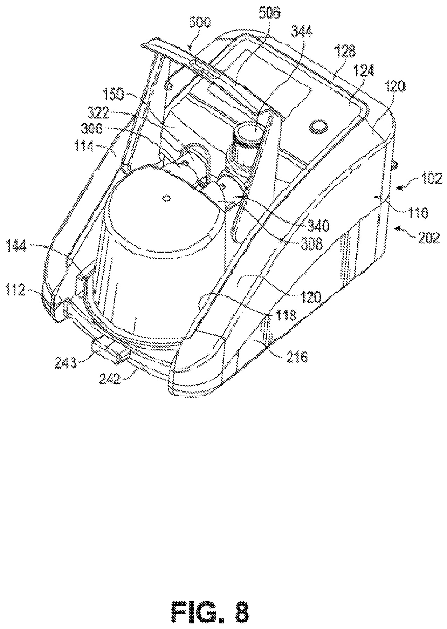

A gases inlet port 340 (humidified gases return) as shown in Figure 8 can include a removable L-shaped elbow. The removable elbow can further include a patient outlet port 344 for coupling to the patient conduit 16 to deliver gases to the patient interface. The gases outlet port 322, gases inlet port 340, and patient outlet port 344 each can have soft seals such as O-ring seals or T-seals to provide a sealed gases passageway between the apparatus 10, the humidification chamber 300, and the patient conduit 16.

The humidification chamber gases inlet port 306 can be complementary with the gases outlet port 322, and the humidification chamber gases outlet port 308 can be complementary with the gases inlet port 340. The axes of those ports can be parallel to each other to enable the humidification chamber 300 to be inserted into the chamber bay 108 in a linear movement.

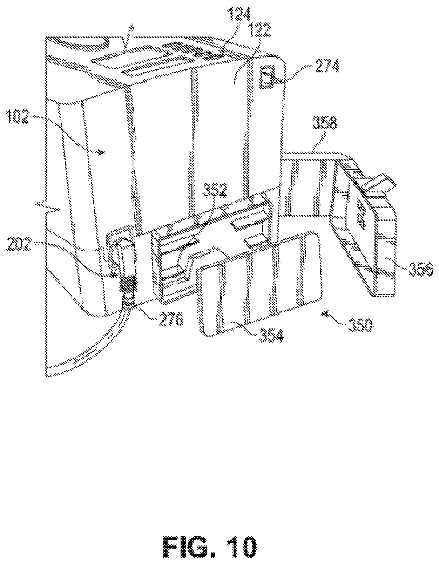

The respiratory device can have air and oxygen (or alternative auxiliary gas) inlets in fluid communication with the motor to enable the motor to deliver air, oxygen (or alternative auxiliary gas), or a mixture thereof to the humidification chamber 300 and thereby to the patient. As shown in Figure 10, the device can have a combined air/oxygen (or alternative auxiliary gas) inlet arrangement 350. This arrangement can include a combined air/oxygen port 352 into the housing 100, a filter 354, and a cover 356 with a hinge 358. A gases tube can also optionally extend laterally or in another appropriate direction and be in fluid communication with an oxygen (or alternative auxiliary gas) source. The port 352 can be fluidly coupled with the motor 402. For example, the port 352 may be coupled with the motor/sensor module 400 via a gases flow passage between the port 352 and an inlet aperture or port in the motor and sensor module 400, which in turn would lead to the motor.

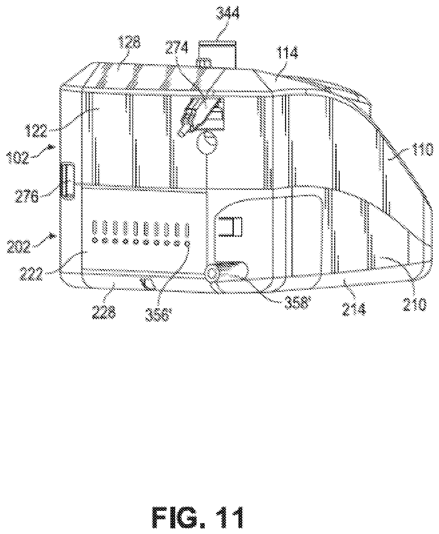

The device can have the arrangement shown in Figures 11 to 14 to enable the blower to deliver air, oxygen (or alternative auxiliary gas), or a suitable mixture thereof to the humidification chamber 300 and thereby to the patient. This arrangement can include an air inlet 356' in the rear wall 222 of the lower chassis 202 of the housing 100. The air inlet 356' comprises a rigid plate with a suitable grill arrangement of apertures and/or slots. Sound dampening foam may be provided adjacent the plate on the interior side of the plate. An air filter box 354' can be positioned adjacent the air inlet 356' internally in the main housing 100, and include an air outlet port 360 to deliver filtered air to the motor via an air inlet port 404 in the motor/sensor module 400. The air filter box 354' may include a filter configured to remove particulates (e.g. dust) and/or pathogens (e.g. viruses or bacteria) from the gases flow. A soft seal such as an O-ring seal can be provided between the air outlet port 360 and air inlet port 404 to seal between the components. The device can include a separate oxygen inlet port 358' positioned adjacent one side of the housing 100 at a rear end thereof, the oxygen port 358' for receipt of oxygen from an oxygen source such as a tank or source of piped oxygen. The oxygen inlet port 358' is in fluid communication with a valve 362. The valve 362 can suitably be a solenoid valve that enables the control of the amount of oxygen that is added to the gases flow that is delivered to the humidification chamber 300. The oxygen port 358' and valve 362 may be used with other auxiliary gases to control the addition of other auxiliary gases to the gases flow. The other auxiliary gases can include any one or more of a number of gases useful for gas therapy, including but not limited to heliox and nitric oxide.

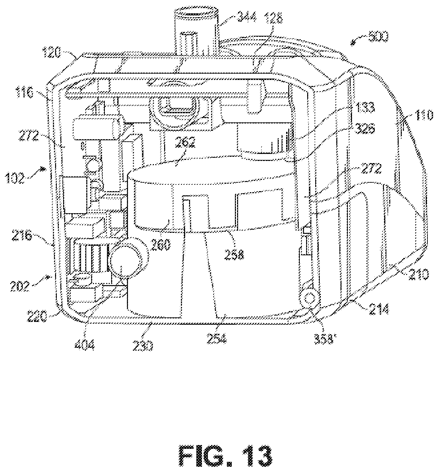

As shown in Figures 13 to 16, the lower housing chassis 202 can include suitable electronics boards, such as sensing circuit boards. The electronics boards can be positioned adjacent respective outer side walls 210, 216 of the lower housing chassis 202. The electronics boards can contain, or can be in electrical communication with, suitable electrical or electronics components, such as but not limited to microprocessors, capacitors, resistors, diodes, operational amplifiers, comparators, and switches. Sensors can be used with the electronic boards. Components of the electronics boards (such as but not limited to one or more microprocessors) can act as the controller 19 of the apparatus.

One or more of the electronics boards can be in electrical communication with the electrical components of the apparatus 10, including the display unit and user interface 54, motor, valve 362, and the heater plate 140 to operate the motor to provide the desired flow rate of gases, operate the humidification chamber 300 to humidify and heat the gases flow to an appropriate level, and supply appropriate quantities of oxygen (or quantities of an alternative auxiliary gas) to the gases flow.

The electronics boards can be in electrical communication with a connector arrangement 274 projecting from the rear wall 122 of the upper housing chassis 102. The connector arrangement 274 may be coupled to an alarm, pulse oximetry port, and/or other suitable accessories. The electronics boards can also be in electrical communication with an electrical connector 276 that can also be provided in the rear wall 122 of the upper housing chassis 102 to provide mains or battery power to the components of the device.

As mentioned above, operation sensors, such as flow, temperature, humidity, and/or pressure sensors can be placed in various locations in the respiratory device, the patient breathing conduit 16, and/or cannula 51 such as shown in Figure 1. The electronics boards can be in electrical communication with those sensors. Output from the sensors can be received by the controller 19, to assist the controller 19 to operate the respiratory apparatus 10 in a manner that provides optimal therapy, for example controlling to a set flow rate. The set flow rate may be selected such that it provides flushing of the patient's upper airways and/or meets or exceeds a patient's inspiratory demand and/or provides other advantages of high flow therapy described herein. In the illustrated embodiment the sensors are positioned on electronic boards that are positioned within the housing. The sensors are encapsulated within the housing.

As outlined above, the electronics boards and other electrical and electronic components can be pneumatically isolated from the gases flow path to improve safety. The sealing also prevents water ingress.

### 1.1 Control System

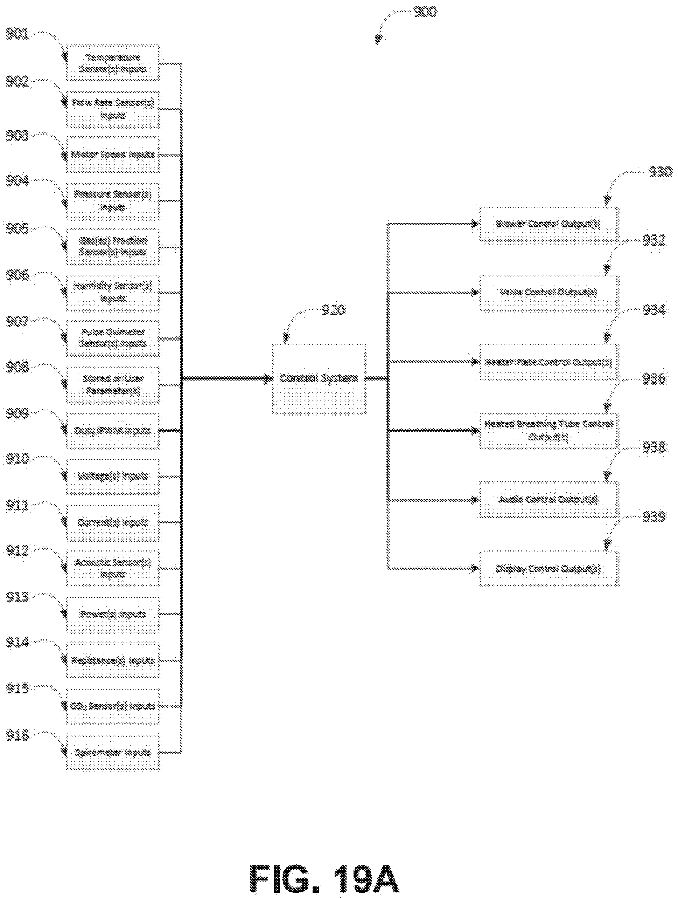

FIG. 19A illustrates a block diagram 900 of an example control system 920 (which can be the controller 19 in Figure 1) that can detect patient conditions and control operation of the respiratory system including the gases source. The control system 920 can manage a flow rate of the gases flowing through the respiratory system as is the gases are delivered to a patient. For example, the control system 920 can increase or decrease the flow rate by controlling an output of a motor speed of the blower (hereinafter also referred to as a "blower motor") 930 or an output of a valve 932 in a blender. The control system 920 can automatically determine a set value or a personalized value of the flow rate for a particular patient as discussed below. The flow rate can be optimized by the control system 920 to improve patient comfort and therapy.

The control system 920 can also generate audio and/or display/visual outputs 938, 939. For example, the flow therapy apparatus can include a display and/or a speaker. The display can indicate to the physicians any warnings or alarms generated by the control system 920. The display can also indicate control parameters that can be adjusted by the physicians. For example, the control system 920 can automatically recommend a flow rate for a particular patient. The control system 920 can also determine a respiratory state of the patient, including but not limited to generating a respiratory rate of the patient, and send it to the display, which will be described in greater detail below.

The control system 920 can change heater control outputs to control one or more of the heating elements (for example, to maintain a temperature set point of the gases delivered to the patient). The control system 920 can also change the operation or duty cycle of the heating elements. The heater control outputs can include heater plate control output(s) 934 and heated breathing tube control output(s) 936.

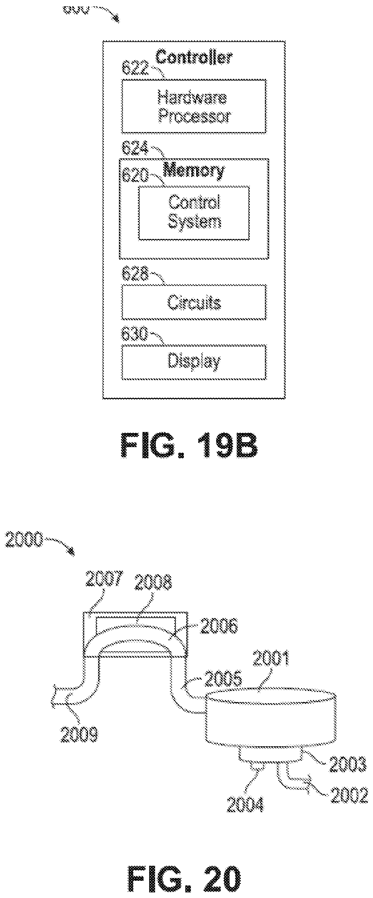

The control system 920 can determine the outputs 930-939 based on one or more received inputs 901-916. The inputs 901-916 can correspond to sensor measurements received automatically by the controller 600 (shown in FIG. 19B). The control system 920 can receive sensor inputs including but not limited to temperature sensor(s) inputs 901, flow rate sensor(s) inputs 902, motor speed inputs 903, pressure sensor(s) inputs 904, gas(s) fraction sensor(s) inputs 905, humidity sensor(s) inputs 906, pulse oximeter (for example, SpO₂) sensor(s) inputs 907, stored or user parameter(s) 908, duty cycle or pulse width modulation (PWM) inputs 909, voltage(s) inputs 910, current(s) inputs 911, acoustic sensor(s) inputs 912, power(s) inputs 913, resistance(s) inputs 914, CO₂ sensor(s) inputs 915, and/or spirometer inputs 916. The control system 920 can receive inputs from the user or stored parameter values in a memory 624 (shown in FIG. 19B). The control system 920 can dynamically adjust flow rate for a patient over the time of their therapy. The control system 920 can continuously detect system parameters and patient parameters. A person of ordinary skill in the art will appreciate based on the disclosure herein that any other suitable inputs and/or outputs can be used with the control system 920.

### 1.2 Controller

Figure 19B illustrates a block diagram of an embodiment of a controller 600 (which can be the controller 19 in Figure 1). The controller 600 can include programming instructions for detection of input conditions and control of output conditions. The programming instructions can be stored in the memory 624 of the controller 600. The programming instructions can correspond to the methods, processes and functions described herein. The programming instructions can be executed by one or more hardware processors 622 of the controller 600. The programming instructions can be implemented in C, C++, JAVA, or any other suitable programming languages. Some or all of the portions of the programming instructions can be implemented in application specific circuitry 628 such as ASICs and FPGAs.

The controller 600 can also include circuits 628 for receiving sensor signals. The controller 600 can further include a display 630 for transmitting status of the patient and the respiratory assistance system. The display 630 can also show warnings and/or other alerts. The display 630 can be configured to display characteristics of sensed gas(es) in real time or otherwise. The controller 600 can also receive user inputs via the user interface such as display 630. The user interface can include button(s) and/or dial(s). The user interface can comprise a touch screen.

### 1.3 Motor and Sensor module

Any of the features of the respiratory system described herein, including but not limited to the humidification chamber, the flow generator, the user interface, the controller, and the patient breathing conduit configured to couple the gases flow outlet of the respiratory system to the patient interface, can be combined with any of the sensor modules described herein.

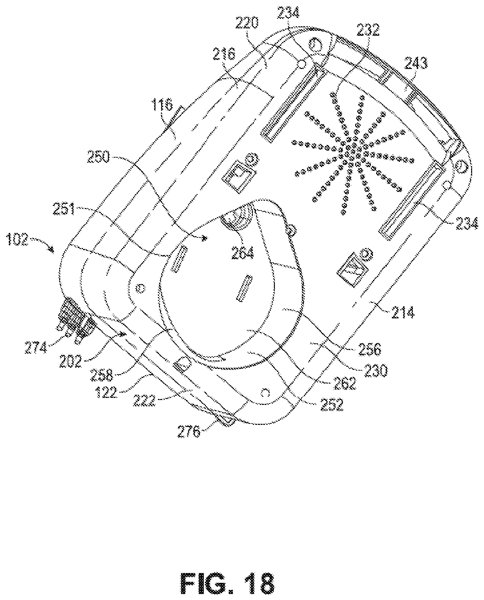

Figure 20 illustrates a block diagram of the motor and sensor module 2000, which can be received by the recess 250 in the respiratory device (shown in Figures 17 and 18). The motor and sensor module can include a blower 2001, which entrains room air to deliver to a patient. The blower 2001 can be a centrifugal blower.

One or more sensors (for example, Hall-effect sensors) may be used to measure a motor speed of the blower motor. The blower motor may comprise a brushless DC motor, from which motor speed can be measured without the use of separate sensors. For example, during operation of a brushless DC motor, back-EMF can be measured from the nonenergized windings of the motor, from which a motor position can be determined, which can in turn be used to calculate a motor speed. In addition, a motor driver may be used to measure motor current, which can be used with the measured motor speed to calculate a motor torque. The blower motor may comprise a low inertia motor.

Room air can enter a room air inlet 2002, which enters the blower 2001 through an inlet port 2003. The inlet port 2003 can include a valve 2004 through which a pressurized gas may enter the blower 2001. The valve 2004 can control a flow of oxygen into the blower 2001. The valve 2004 can be any type of valve, including a proportional valve or a binary valve. In some embodiments, the inlet port does not include a valve.

The blower 2001 can operate at a motor speed of greater than 1,000 RPM and less than 30,000 RPM, greater than 2,000 RPM and less than 21,000 RPM, or between any of the foregoing values. Operation of the blower 2001 mixes the gases entering the blower 2001 through the inlet port 2003. Using the blower 2001 as the mixer can decrease the pressure drop that would otherwise occur in a system with a separate mixer, such as a static mixer comprising baffles, because mixing requires energy.

The mixed air can exit the blower 2001 through a conduit 2005 and enters the flow path 2006 in the sensor chamber 2007. A sensing circuit board with sensors 2008 can positioned in the sensor chamber 2007 such that the sensing circuit board is at least partially immersed in the gases flow. At least some of the sensors 2008 on the sensing circuit board can be positioned within the gases flow to measure gases properties within the flow. After passing through the flow path 2006 in the sensor chamber 2007, the gases can exit 2009 to the humidification chamber.

Positioning sensors 2008 downstream of the combined blower and mixer 2001 can increase accuracy of measurements, such as the measurement of gases fraction concentration, including oxygen concentration, over systems that position the sensors upstream of the blower and/or the mixer. Such a positioning can give a repeatable flow profile. Further, positioning the sensors downstream of the combined blower and mixer avoids the pressure drop that would otherwise occur, as where sensing occurs prior to the blower, a separate mixer, such as a static mixer with baffles, is required between the inlet and the sensing system. The mixer can introduce a pressure drop across the mixer. Positioning the sensing after the blower can allow the blower to be a mixer, and while a static mixer would lower pressure, in contrast, a blower increases pressure. Also, immersing at least part of the sensing circuit board and sensors 2008 in the flow path can increase the accuracy of measurements because the sensors being immersed in the flow means they are more likely to be subject to the same conditions, such as temperature and pressure, as the gases flow and therefore provide a better representation of the gases flow characteristics.

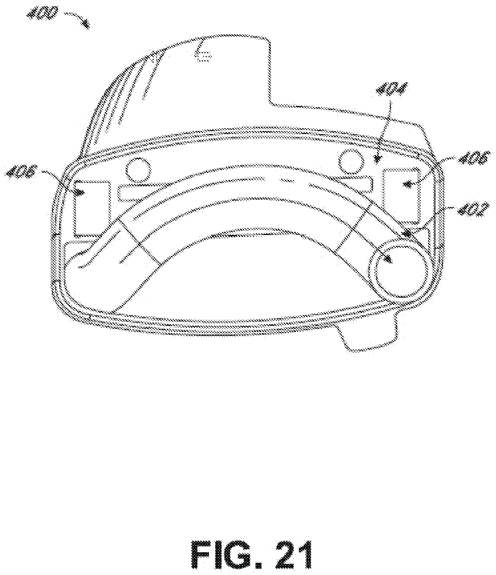

Referring to Figure 21, the gases exiting the blower can enter a flow path 402 in a sensor chamber 400, which can be positioned within the motor and sensor module and can be the sensor chamber 2007 of Figure 20. The flow path 402 can have a curved shape. The flow path 402 can be configured to have a curved shape with no sharp turns. The flow path 402 can have curved ends with a straighter section between the curved ends. A curved flow path shape can reduce pressure drop in a gases flow without reducing the sensitivity of flow measurements by partially coinciding a measuring region with the flow path to form a measurement portion of the flow path.

A sensing circuit board 404 with sensors, such as acoustic transmitters and/or receivers, humidity sensor, temperature sensor, thermistor, and the like, can be positioned in the sensor chamber 400 such that the sensing circuit board 404 is at least partially immersed in the flow path 402. Immersing at least part of the sensing circuit board and sensors in the flow path can increase the accuracy of measurements because the sensors immersed in the flow are more likely to be subject to the same conditions, such as temperature and pressure, as the gases flow, and therefore provide a better representation of the characteristics of the gases flow. After passing through the flow path 402 in the sensor chamber 400, the gases can exit to the humidification chamber.

The gases flow rate may be measured using at least two different types of sensors. The first type of sensor can comprise a thermistor, which can determine a flow rate by monitoring heat transfer between the gases flow and the thermistor. The thermistor flow sensor can run the thermistor at a constant target temperature within the flow when the gases flow around and past the thermistor. The sensor can measure an amount of power required to maintain the thermistor at the target temperature. The target temperature can be configured to be higher than a temperature of the gases flow, such that more power is required to maintain the thermistor at the target temperature at a higher flow rate.

The thermistor flow rate sensor can also maintain a plurality of (for example, two, three, or more) constant temperatures on a thermistor to avoid the difference between the target temperature and the gases flow temperature from being too small or too large. The plurality of different target temperatures can allow the thermistor flow rate sensor to be accurate across a large temperature range of the gases. For example, the thermistor circuit can be configured to be able to switch between two different target temperatures, such that the temperature of the gases flow will always fall within a certain range relative to one of the two target temperatures (for example, not too close but not too far). The thermistor circuit can be configured to operate at a first target temperature of about 50°C to about 70°C, or about 66°C. The first target temperature can be associated with a desirable flow temperature range of between about 0⁰C to about 60°C, or about 0°C and about 40°C. The thermistor circuit can be configured to operate at a second target temperature of about 90°C to about 110°C, or about 100°C. The second target temperature can be associated with a desirable flow temperature range of between about 20°C to about 100°C, or about 30°C and about 70°C.

The controller can be configured to adjust the thermistor circuit to change between at least the first and second target temperature modes by connecting or bypassing a resistor within the thermistor circuit. The thermistor circuit can be arranged as a Wheatstone bridge configuration comprising a first voltage divider arm and a second voltage divider arm. The thermistor can be located on one of the voltage divider arms. More details of a thermistor flow rate sensor are described in PCT Application Publication No. WO2018/052320, filed 3 September 2017.

The second type of sensor can comprise an acoustic sensor assembly. Acoustic sensors including acoustic transmitters and/or receivers can be used to measure a time of flight of acoustic signals to determine gases velocity and/or composition, which can be used in flow therapy apparatuses. In one ultrasonic sensing (including ultrasonic transmitters and/or receivers) topology, a driver causes a first sensor, such as an ultrasonic transducer, to produce an ultrasonic pulse in a first direction. A second sensor, such as a second ultrasonic transducer, receives this pulse and provides a measurement of the time of flight of the pulse between the first and second ultrasonic transducers. Using this time of flight measurement, the speed of sound of the gases flow between the ultrasonic transducers can be calculated by a processor or controller of the respiratory system. The second sensor can transmit and the first sensor can receive a pulse in a second direction opposite the first direction to provide a second measurement of the time of flight, allowing characteristics of the gases flow, such as a flow rate or velocity, to be determined. In another acoustic sensing topology, acoustic pulses transmitted by an acoustic transmitter, such as an ultrasonic transducer, can be received by acoustic receivers, such as microphones. More details of an acoustic flow rate sensor are described in PCT Application Publication No. WO2017/095241, filed 2 December 2016.

The one or more flow rate sensors, or a sensor assembly comprising a flow rate sensor or sensors, may be located in various positions in the respiratory apparatus and/or along the gases flow path. In one configuration, a flow rate sensor or sensors, or a sensory assembly, may be located or arranged after the flow generator 50B, i.e. the sensor is configured or arranged to sense or measure the flow rate of the gases in the flow path after the flow generator 50B. In this configuration, the flow rate signal or flow rate data generated by the flow rate sensor or sensors may represent the flow generator output flow rate signal or data, i.e. the flow rate of the gases flow output from the flow generator 50B.

In one example configuration, a flow rate sensor or sensors, or sensor assembly, may be located in the main device housing 100 before or after the humidifier 52 (if present). For example, the flow rate sensor may be arranged or configured in the main device housing 100 to sense the flow rate of the gases in the flow path at a location between the flow generator 50B and humidifier 52, or a location in the flow path after the humidifier. In another example configuration, a flow rate sensor or sensors, or sensor assembly, may be located in or along the breathing conduit 16 and/or patient interface 51. In this configuration, the sensor or sensor assembly is configured to sense or measure the flow rate of the gases flow in the flow path comprising or formed by the breathing conduit 16 and/or patient interface 51, i.e. the flow path that follows the gases flow outlet 21 of the main device housing 100. In another example configuration, the apparatus may comprise any combination of the mentioned one or more flow rate sensor or sensor assembly configurations or locations. For example, the apparatus may comprise any combination of one or more flow rate sensors or sensor assemblies in any one or more locations along the gases flow path, whether in the main device housing 100, breathing conduit 16, and/or patient interface 51.

In some configurations, readings from both the first and second types of sensors can be combined to determine a more accurate flow measurement. For example, a previously determined flow rate and one or more outputs from one of the types of sensor can be used to determine a predicted current flow rate. The predicted current flow rate can then be updated using one or more outputs from the other one of the first and second types of sensor, in order to calculate a final flow rate.

### 2. Example embodiment of breathing parameter determination processes

The methods and processes of determining the breathing parameter estimates and/or ratios relating to or indicative of inspiration time, expiration time, and/or total respiration time, will be described in the context of the example respiratory apparatus 10 described above, which is configured or operable to provide nasal high flow therapy via an unsealed patient interface. As explained earlier, the methods and processes may also be applied to other respiratory apparatus and/or to other modes of operation and/or modes of therapy delivered by such apparatus.

The present disclosure relates to a respiratory apparatus that is configured to provide a flow of gases to a user for respiratory therapy comprising: a flow generator that is operable to generate a flow of gases; a controller that is operatively connected to the flow generator and operable to control a flow rate of the flow of gases by controlling the flow generator, wherein the controller is configured during operation to: receive flow rate data indicative of or representing the flow rate of the flow of gases; process the flow rate data to extract or generate breathing data indicative of or representing the patient's breathing or respiration from the flow rate data; and process the breathing data to calculate one or more primary breathing parameter ratios representative of the ratio between inspiration time and/or expiration time to total respiration time for the patient's breathing cycle. One or more of the breathing parameter ratios may be presented on a graphical user interface. The ratio may be plotted graphically or may be an alphanumeric value that is presented on a graphical user interface.

### 2.1 Summary of breathing parameter determination processes

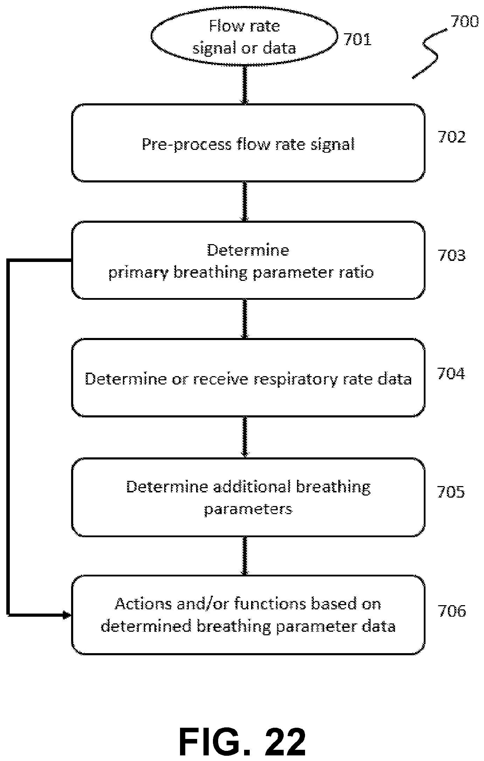

Referring to Figure 22, a summary flow diagram of an embodiment of the breathing parameter process or algorithm 700 in a first configuration is shown. The algorithm 700 operates or executes during operation of the respiratory apparatus 10, i.e. when it is delivering high flow therapy to a patient.

At step 701, the algorithm 700 receives or retrieves flow parameter data such as, but not limited to, a 'raw' flow rate signal or flow rate data e.g. from one or more flow rate sensors of the respiratory apparatus, representing or indicative of the flow rate of the flow of gases or gases stream delivered to the patient. In this example, the algorithm operates continuously on new flow rate data as it arrives and processes the arriving data in the following manner.

At step 702, the raw flow rate data is pre-processed, e.g. filtered or otherwise processed, to remove unwanted signal components, such as those present from the flow generator motor. The output of the pre-processing is a signal representative or indicative of patient breathing data (with some residual noise component). In one configuration, the pre-processed signal may be in the form of flow parameter variation data. Optionally, this pre-processing step 702 may comprise a preliminary stage of assessing the quality of the incoming raw flow rate data prior to further pre-processing. For example, good quality raw flow rate data may be further pre-processed into the flow parameter variation data and passed to the next step, but poor-quality data may be discarded.

At step 703, the pre-processed flow parameter variation data is processed to determine or calculate a new or updated primary breathing parameter ratio representing a ratio of the inspiratory time and/or expiratory time to the total respiratory time. The breathing parameter ratio may optionally be calculated as a rolling average that is summed. The ratio may be summed and stored. The stored rolling average of the breathing parameter ratio may be presented on a display (i.e. a graphical user interface).

At step 704, the algorithm 700 may optionally either receive data indicative of the current respiratory rate or calculate respiratory rate data representing the current respiratory rate based on the pre-processed data from step 702. Determining the respiratory rate at step 704 may be carried out before, in parallel, or after step 703.

At step 705, the algorithm 700 may optionally determine one or more additional breathing parameters and/or ratios based on the calculated breathing parameter ratio from step 703 and the respiratory rate data from step 704.

As mentioned above, as new flow rate data arrives, the algorithm continues to execute and update the parameters being calculated, such that the parameters and/or ratios are updated in real-time as the respiratory apparatus is in operation and delivering high flow therapy.

At step 706, the algorithm 700 may execute one or more actions and/or functions based on the calculated or updated primary breathing parameter ratio from step 703 and/or additional breathing parameters from step 705. By way of example only, the breathing parameter data generated by the algorithm during operation of the respiratory apparatus may be continuously fed to other control functions of the apparatus for the purpose of analysis, monitoring, display, alarms, storage, and/or notifications.

### 2.2 Detailed overview of breathing parameter determination processes

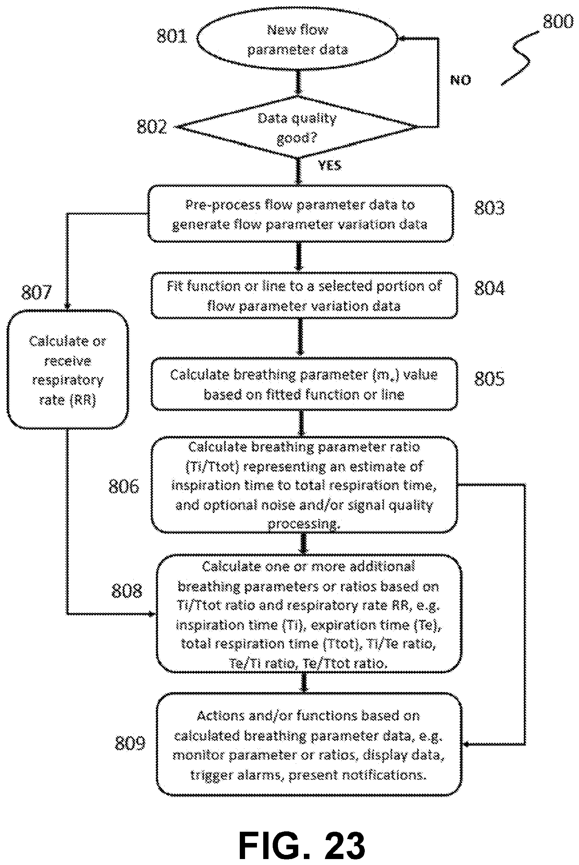

Referring to Figure 23, a detailed flow diagram of an embodiment of the breathing parameter process or algorithm 800 in a second configuration is shown. The algorithm 800 operates in a similar manner to the summary of algorithm 700.

At step 801, algorithm 800 receives or retrieves new flow parameter data. By way of example, the flow parameter data may be flow rate signal data or data indicative or representative of the total flow rate of the gases stream in the respiratory apparatus. As will be appreciated, the flow parameter data may be received or retrieved from one or more flow rate sensors or sensor assemblies in the gases flow path of the respiratory apparatus. As previously described above, the flow rate sensor or sensor assemblies of the respiratory apparatus may be any suitable type of flow rate sensor including, but not limited to, thermistor flow rate sensors and/or acoustic or ultrasonic-based flow rate sensors or sensor assemblies. In embodiment, the flow rate sensors are located or configured to sense the flow rate of the gases flow in the flow path after the blower or flow generator of the respiratory apparatus.

At step 802, the incoming new flow parameter data is evaluated or assessed to determine if it is of sufficient quality for further processing. For example, the data will be of poor quality for further processing if it contains large signal variations, e.g. such as large transient peaks caused by the patient adjusting their interface (e.g. nasal cannula), for example. If the data is of poor quality, the algorithm discards the data and returns to the start 801 to process the next new incoming flow parameter data. If the data is of good quality, the algorithm progresses to the next step 803. The controller assesses the quality of the incoming flow parameter data. Alternatively, the controller may use variations in flow conductance to assess the quality of the flow parameter data.

At step 803, the 'good quality' flow parameter data is pre-processed to remove unwanted signal components (such as those generated by the flow generator) and to generate flow parameter variation data, which is representative of the breathing data (and residual noise) of the patient.

In one configuration, at step 804, the algorithm fits a function or line to the flow parameter variation data generated at step 803. For example, the algorithm fits a function or line to a selected portion of the flow parameter variation data. At step 805, the algorithm then calculates a breathing parameter value (*m*₊) based on the fitted function or line.

In another configuration, the algorithm does not perform step 804, and proceeds to calculate the breathing parameter value (*m*₊) at step 805 directly from the flow parameter variation data representing the patient's breathing.

The breathing parameter value (*m*₊) generated in step 805 is a value that represents or is indicative of whether the patient is currently inspiring or expiring. In this embodiment, the breathing parameter value (*m*₊) is a Boolean value or truth value that changes depending on the patient's breathing, i.e. whether they are inspiring or expiring. In this example configuration, the breathing parameter value (*m₊*) is a Boolean value that represents or is indicative of whether the patient is currently inspiring. For example, in this embodiment, the controller is configured to assign a 1 value if the patient is inspiring i.e. (*m*₊) is 1 for patient inspiration, and the controller is configured to assign a 0 when the patient is expiring i.e. (*m*₊) is 0 for patient expiration. The inspiration and expiration are determined based on the fitted function or line of the flow parameter variation data from step 804 or directly from the flow parameter variation data, depending on the configuration. The data processing, and associated criteria or threshold(s), applied to determine the breathing parameter value will be explained in further detail later.

At step 806, the algorithm calculates a rolling or running average or filtered average of the breathing parameter value (*m*₊) calculated from the prior steps. This rolling average value is representative or indicative of the current breathing parameter ratio *(Ti*/*Ttot)* of the inspiration time *(Ti)* to the total respiration time *(Ttot)* of the patient's breathing or respiration cycle. Optionally, in some configurations, this step 806 may comprise or involve one or more additional processes or processing stages. As will be explained further, such optional additional stages may, for example, include: noise correction and/or noise cleaning and/or noise filtering processes; and/or signal or data quality determination or processing.

At step 807, the algorithm is configured to receive, retrieve or calculate a real-time value or parameter representing or indicative of the current respiratory rate of the patient. The respiratory rate data can be received from other sources or sensors, or alternatively may be calculated from the flow parameter data or flow parameter variation data.

At step 808, the algorithm may calculate one or more additional breathing parameters or ratios based on the breathing parameter ratio representing *Ti*/*Ttot* and the respiratory rate data. Such additional breathing parameters or ratios may include, for example, inspiration time *(Ti),* expiration time *(Te),* total respiration time *(Ttot),* ratio of inspiration time to expiration time *(Ti*/*Te),* ratio of expiration time to inspiration time *(Te*/*Ti),* and/or ratio of expiration time to total respiration time *(Te*/*Ttot).*

As will be appreciated, as new flow rate data arrives and is processed by the algorithm 800, the algorithm continues to update the breathing parameters being calculated, such that the parameters and/or ratios are updated in real-time as the respiratory apparatus is in operation and delivering therapy. The breathing parameter data may be fed to other functions or operations of the controller, as explained further below.

At step 809, the algorithm may execute one or more actions and/or functions based on the calculated or updated breathing parameter data such as, but not limited to, monitoring trends in the data, presenting or displaying the data on the display of the apparatus, triggering alarms based on the data, presenting or displaying notifications based on the data, presenting or displaying suggestions or prompts for therapy parameter or setting changes (e.g. 'increase flow rate'), and/or updating or automatically varying apparatus operation or therapy settings (e.g. flow rate) based on the data.

In this example configuration, the incoming data and processed data generated and/or output by the algorithm 800 is stored in memory over time as required, including data such as, but not limited to, the incoming flow parameter data, flow parameter variation data, fitted function or line data, breathing parameter value (*m₊*), breathing parameter ratio(s) or breathing parameters (e.g. *Ti*/*Ttot, Te*/*Ttot, Ti*/*Te, Te*/*Ti, Ti, Te, Ttot),* and respiratory rate data for example. Such data or at least required portions of the raw or processed data received or generated from any of the stages of the algorithm may be stored in memory as time-series data, as this enables data variables to be tracked and/or further processing or filtering of any one of more of the data variables or data parameters according to one or more functions and/or generating one or more statistical parameters based on at least a portion of the stored time-series data, such as moving or running averages or the like.

Each of the steps 801-809 of the example algorithm 800 will now be explained in further detail under sections 2.3-2.7 below. To the extent that algorithm 700 has the same or similar steps or processes, the explanation in relation to algorithm 800 may also apply to algorithm 700 in some embodiments. It will be appreciated that algorithm 800 is one example configuration for deriving breathing parameter data. However, variations or modifications can be made to the algorithm depending on application requirements. Not all process steps may be necessary in all variations of the algorithm, depending on the breathing parameter data required and application of the algorithm. In other embodiments, depending on requirements, one or more of the steps and/or stages of the algorithm may be combined or further separated, re-ordered, omitted and/or modified to suit the design requirements. The flow diagrams discussed and explained are for assisting the explanation of the algorithm processes and principles.

### 2.3 Pre-processing of flow rate signal or flow rate data

Steps 801-803 of algorithm 800 relate to receiving and pre-processing new flow parameter data, and example processes will be explained in further detail below.

When a patient is breathing through his or her nose into the patient interface of the respiratory apparatus, a breathing signal is detected in the flow rate or other flow parameters due to the flow resistance variation caused by inhalation and exhalation.

The flow rate or other gases flow parameter signal can be fed through a pre-processing step or steps (e.g. 802, 803). This step or steps may allow the controller to decide whether the gases flow parameter data is suitable for use in determining patient breathing parameters, and/or to remove certain features from the flow parameters, such that the flow parameter signal that is fed into the remaining breathing parameter determination process can be more representative of any effects the patient's respiration is having on the gases flow parameter (such as the flow rate, pressure, or otherwise).

As described above, it is assumed that fluctuations in the pre-processed flow rate or other flow parameter data are made up of random uncorrelated noise and a correlated breathing signal generated by the patient, if the patient is attached to the respiratory system and breathing through the patient interface.

As discussed above, the flow data in an unsealed system, such as nasal high flow systems, can be difficult to determine. The open nature of the system results in a very low signal to noise ratio. Any flow data measured by the sensor(s) can include various irregularities and noise that can obscure the flow data that is informative of the device and/or patient breath flows and which must be accounted for to accurately determine the desired measurement.

In order to remove noise and other irregularities from any obtained flow data, the flow signal can be fed through a pre-processing step or steps (e.g. 802, 803). Pre-processing can allow the controller to remove certain distortions from the flow parameters, such that the flow parameter signal that is used to determine the breathing parameters can better reflect the effect the gas flow parameter used in the patient's treatment is having on the patient's respiration.

If the patient is attached to the respiratory system and breathing through the patient interface, fluctuations in pre-processed flow rate or other flow parameter data obtained in an open system are made up of random uncorrelated noise (which comes from various sources, not from the patient) and a correlated breathing signal generated by the patient. The pre-processing of the data can start with the controller receiving the flow parameter data (such as unprocessed data), as shown at step 801. The controller can then perform an initial pre-processing step, for example, by determining if the flow parameter data is good or suitable for use, as shown at step 802. If the data is not suitable for use, the controller can discard the data, and return to step 801 awaiting the next new flow parameter data, as shown.

In determining the suitability of the data, the controller can receive second flow parameter data that is of a different type than a first flow parameter data. The second flow parameter data is assumed to have some correlation to the first parameter. The second flow parameter data can include, for example, the motor speed, pressure, and/or oxygen flow rate or concentration or any other parameter that can have an effect on or provide an indication of the gases flow rate that is separate from the effect of the patient's respiration on the gases flow rate. The controller can be configured to determine whether the second flow parameter data is useful as a correlation parameter to the first flow parameter data. For example, the second flow parameter data can be a useful correlation metric if the second flow parameter data meets a threshold level. If the second flow parameter data does not meet a threshold level, then it is assumed the second flow parameter data is not correlated to the first flow parameter data. As such, the second flow parameter data can be ignored or thrown out. If there is insufficient second flow parameter data, the controller can determine that it has insufficient data to use the first flow parameter data and may discard the first parameter data. If the second flow parameter data meets a minimum threshold level, the controller can determine that the first parameter data is suitable for use.

As an example, the second flow parameter data can represent the motor speed. In order to identify the patient's respiration in the first flow parameter data, the motor needs to be operating at a sufficient speed. If the motor speed is too low, the effect or correlation of the motor speed on the flow data (such as the flow rate) may not be accurately predictable. Therefore, after the controller has received the motor speed data, the controller can compare the motor speed to a minimum motor speed threshold. If the motor speed is below the threshold, the controller can deem the first flow parameter data as unsuitable and can discard a portion or all of the first flow parameter data. However, if the motor speed is above the threshold, the controller can calculate the recent changes in the motor speed. A change in motor speed can result in a change in the first flow parameter data, which makes it more difficult to identify the patient's respiration in the first flow parameter data. While the effect of the motor speed can be removed from the first flow parameter data to some degree, larger changes in motor speed may make the data too unreliable for identifying the patient's respiration. Therefore, the controller can apply a running filter to the relative changes in motor speed in order to generate a first value representing the recent relative changes in motor speed. The controller can then compare the first value with a first threshold. If the first value is above the first threshold, the controller can deem the flow parameter data to be unsuitable, and the flow data point can be discarded. If the first value is below the first threshold, the controller can deem the flow parameter data to be suitable for use.

As another example, the second flow parameter data can represent the concentration of a supplementary gas from a supplementary gas source. The first flow parameter data (such as the flow rate) can be affected by the flow rate or concentration of supplementary gas from a supplementary gas source. The controller can receive an oxygen flow rate data or an oxygen concentration data. The controller can calculate recent changes in the oxygen flow rate or the oxygen concentration. If the flow rate or concentration of oxygen changes, the resulting change in the total flow rate can make it more difficult to identify the patient's respiration in the flow rate signal or other flow parameter signal. The controller can therefore apply a running filter to the changes in oxygen concentration of the gases or the oxygen flow rate in order to generate a second value representing the recent changes in oxygen concentration or flow rate. The controller can compare the second value with a second threshold. If the second value is above the second threshold, the controller can determine the first flow parameter data is unsuitable, and the first flow parameter data point can be discarded. However, if the second flow parameter data is below the threshold, the controller can deem the flow parameter data to be suitable.

As described above, if the controller deems the data to be suitable, the first flow parameter data (or any other flow parameter data) can also be modified or further pre-processed to remove the effect of the motor (or other factors, such as the oxygen concentration or flow rate) as shown at step 803, to generate the flow parameter variation data. Modifying the first flow parameter data can involve removing the assumed effect of other variables from the first flow parameter data (such as the motor speed). This assumed effect is only valid if the gases flow parameter data meets certain criteria. As described above, if these criteria are not met, the data may be discarded.

The process can modify the first flow parameter data to remove the effect of motor speed. The effect of the motor can be estimated using the motor speed and the flow conductance. The controller can measure an instantaneous flow conductance. In one configuration, a filtered flow conductance can be determined using the following equation: $C = filt \left(\frac{Q}{{ω}_{motor}}\right)$

Where C is the filtered flow conductance, filt() is a filter function (preferably a low-pass filter), Q is the flow parameter data (optionally/preferably a flow rate signal generated by the device flow sensor) and ωₘₒₜₒᵣ is the motor speed. The flow conductance is approximately constant with time, and can therefore be estimated using a low pass filter. The controller measures the instantaneous flow conductance at each iteration using the current motor speed and a measured flow rate. The controller can filter the instantaneous flow conductance in order to determine a filtered flow conductance.

The controller can compare the instantaneous flow conductance with the filtered flow conductance to see if the difference is significantly different. If the difference is significant, it is likely that something has changed the physical system, such as the cannula being attached or detached. The instantaneous flow conductance can be compared with the filtered flow conductance by taking the difference of the two variables and comparing it with a minimum or a maximum threshold. If the difference exceeds or falls below the threshold, the difference is considered to be significant, and the controller can reset the filtered flow conductance. The controller can also vary the filter coefficient of the filter function in the filtered flow conductance calculation based on the difference between the instantaneous flow conductance and the filtered flow conductance. This allows the filtered flow conductance to change more quickly when the variance of the flow conductance is high, such as when the cannula has first been attached.

If the difference between the instantaneous flow conductance and the filtered flow conductance does not exceed the threshold, the difference is considered to be not significant, and the controller can estimate the effect of the motor on the flow rate. The controller can output a value of the effect using the filtered flow conductance and the motor speed. The value can be subtracted or otherwise removed from the flow rate data to arrive at the pre-processed flow rate data (e.g. flow parameter variation data). The pre-processed flow rate data can be more indicative of the patient's respiratory flow (although the pre-processed flow rate data can still include signal noise).

### 2.4 Determining primary breathing parameter ratio from pre-processed flow rate signal or flow rate data

Steps 804-806 of algorithm 800 relate to receiving the pre-processed flow parameter data and further processing it to obtain an updated real-time primary breathing parameter ratio that directly or indirectly represents or is indicative of the patient's current inspiration time relative to total respiration time or current inspiration time relative to expiration time. In the illustrated method steps 804-806 obtain an updated real time primary breathing parameter ratio that represents or is indicative of a patient's current inspiratory time relative to the total respiration time of the patient. Example processes for steps 804-806 will now be explained in further detail below.

Step 804 in algorithm 800 relates to fitting a function or line to a selected portion of the incoming flow parameter data generated or output from the prior pre-processing steps or stages 801-803 in the algorithm.

As previously described, with reference to steps 801-803, the algorithm starts with the controller receiving the data of a flow parameter (which can include unprocessed data of a first parameter or second parameter) at step 801. The flow parameter can be flow rate or a parameter indicative of flow rate. In a configuration, the flow rate can refer to a total flow rate, including respiratory flow rate, supplemental gases flow rate, or others. In a configuration, the flow rate can refer to the apparatus or device flow rate, which represents the flow rate of the total flow of gases exiting the apparatus (e.g. at the gases outlet port or patient interface) and/or the flow rate of the total flow of gases in the flow path downstream of the flow generator. In a configuration, the flow parameter can be a direct measure of gases flow. The flow parameter can be a pressure (e.g. measured or sensed at one or more locations along the flow path of the apparatus or system), motor speed, or other types of parameters disclosed herein. The flow parameter can be a measure of or parameter indicative of a pressure, motor speed, or other types of parameters disclosed herein. The flow parameter can be representative of performance of a component of the device. Preferably the flow parameter used is the device flow rate. Device flow rate may be determined by a flow sensor configured to measure flow rate of the gases. Alternatively, the device flow rate may be determined based on the measured motor speed or based on the pressure of the gases as described above.

At decision step 802, the controller can perform the pre-processing step, such as by determining if the flow parameter data is good or suitable for use. If the data is not suitable for use, the controller can discard the data at step and return to step 801.

At step 803, the controller can generate flow parameter variation data. The flow parameter variation data can be determined by subtracting a target value of the flow parameter data from the measured value of the flow parameter data. The flow parameter variation data can be determined by subtracting an estimated effect of a second parameter from a measured value of a first parameter. In a configuration, the first flow parameter or first parameter is a device output gases flow rate or a parameter indicative of a gases flow rate. In a configuration, the second flow parameter or second parameter is a measure of, or a parameter indicative of pressure, motor speed, or another flow. The estimated effect of the second parameter on the first parameter can be a change in flow rate that can be expected based on the current value of the second parameter, such as the current motor speed. This estimated effect can assume no noise or patient interaction. The estimated effect can be calculated using the current value of the second parameter, such as the current motor speed, as well as a running average of a relationship between the motor speed and flow rate, which can be used to characterize the relationship between the first flow parameter and the second flow parameter. In a configuration, the flow parameter variation data can be determined by subtracting a first average value of the flow parameter data from a second average value of the flow parameter data. The first average value can be later in time than the second average value. The first average value can also be based on a longer window of data than the second average value. In a configuration, the second average value can be based on a longer window of data than the first average value. The windows of data can be mutually exclusive in time or overlapping in time. The windows of data can relate to the same length of time or different lengths of time. The first average value of the flow parameter data can be determined by applying a filter or ongoing filter to the flow parameter data. The first average value of the flow parameter data can be constantly or continuously updated. The second average value can be based on measured values. In one configuration, the flow parameter variation data (e.g. at step 803 can be calculated after determining that the flow parameter data is suitable for use (e.g. at step 802), as shown in Figure 23. In another configuration, the flow parameter variation data can be calculated before determining if the flow parameter data is suitable for use.

At step 804, the controller selects a portion of the flow parameter variation data to analyze. The portion of the flow parameter variation data selected can be the last measured flow parameter variation data, or flow parameter variation data measured contemporaneously or close in time with the analysis. The portion of the flow parameter variation data can relate to a time period within a predefined time period. The portion can be selected to obtain a data set representing or relating to a specific length of time. Selecting a portion of the processed flow parameter data relating to a longer period of time can result in more noise reliably being filtered out of the processed flow parameter data compared to selecting a portion of the processed flow parameter data relating to a shorter period of time. For example, transient noise/fluctuations may have a lesser influence on the flow parameter data if a wider window of data is analyzed, as the noise may have an 'averaging-out' effect. However, selecting a portion of the processed flow parameter data relating to a longer period of time can result in filtering out breathing signals with higher frequencies compared to selecting a portion of the processed flow parameter data relating to a shorter period of time. Accordingly, there can be a tradeoff between filtering noise and detecting or capturing instantaneous changes when selecting a portion of the processed flow parameter data representing a length of time. In a configuration, it can be advantageous to select a portion of the processed flow parameter data representing a length of time that is less than a breathing period. In a configuration, selecting a portion of the processed flow parameter data representing in the range of 0.5-2 seconds can provide reliability in detecting patient interaction or attachment for the majority of expected breathing frequencies (as well as talking, coughing, etc.), while being a length that makes it less likely to generate a false determination of patient attachment or interaction due to random noise. In a configuration, the selected portion of the processed flow parameter data can be less than 0.5, 0.5-1, 1-1.5, 1.5-2, 2-2.5, 2.5-3, 3-3.5, 3.5-4, 4-4.5, 4.5-5, 5-5.5, 5.5-6, or more than 6 seconds.

In one configuration, the controller (or algorithm executing on the controller) selects a portion of the processed flow parameter data before generating the flow parameter variation data at step 803. In another configuration, the controller selects a portion (or window) of the processed flow parameter data relating to a specific length of time such that the signal noise is filtered out of the measure of instantaneous patient ventilation, described hereafter. In a configuration, the controller selects a portion of the processed flow parameter data relating to a specific length of time such that expected breathing frequencies, which can include all expected breathing frequencies, result in an increased measure of instantaneous patient ventilation.

At step 804, the algorithm fits one or more functions to the selected portion of the flow parameter variation data. The one or more functions can be algebraic, such as polynomial (for example, constant, linear, non-linear, quadratic, cubic, etc.), rational, root, and/or others. The one or more functions can be transcendental, such as exponential, hyperbolic, logarithmic, power, periodic (for example, trigonometric, etc.), and/or others. The controller can perform a variety of line and/or curve fitting techniques to fit the one or more functions to the selected portion of the flow parameter variation data, which can include the non-limiting example techniques of regression analysis, interpolation, extrapolation, linear least squares, non-linear least squares, total least squares, simple linear regressions, robust simple linear regression, polynomial regression, orthogonal regression, Deming regression, linear segmented regression, regression dilution, and/or others. The one or more functions, which includes at least those above, can generate a curve. The curve can be a line. The lines or curves described herein can include a plurality of curves, vertices, and/or other features. The lines described herein can be straight, angled, and/or horizontal. The lines described herein can be a line of best fit.

**In** a configuration, at step 804, the algorithm can perform a least squares fit of a line, which can include fitting a linear function such as a straight line, to the selected portion of flow parameter variation data. For example, a straight line can be represented by: ${\hat{u}}_{fit} = m + st *$ where *m* is the mean value of the line, *s* is the slope, and t* is a linearly increasing normalized time parameter. In a configuration, t* can be a linearly increasing normalized time parameter that is equal to minus one at the oldest data point used and equal to one at the most recent data points used. In another configuration, the controller can fit a horizontal line to the selected portion of flow parameter variation data. The horizontal line can be the average of the flow parameter variation data representing or relating to the selected portion. For example, the horizontal line can be represented by *û_{fit} =* m, where *m* is the mean value. In a configuration, *m* is the mean value of the fitted line over a specific number of discrete data points of the flow parameter variation data. The specific number of discrete data points required or used depends on the selected or desired portion or window of flow parameter variation data to be processed in each iteration and the sampling rate or frequency associated with the incoming the flow parameter data. For example, in one configuration, 20 data points are captured and processed in order to apply line-fitting to a 1-second window of flow parameter variation data, based on a 20Hz sampling rate.

In a first configuration, at step 805, the algorithm calculates a breathing parameter value m+ based on the fitted function or line determined for the current portion or selection of flow parameter variation data. In one form of this first configuration, the breathing parameter value *m*₊ may be a Boolean or binary value or variable or Boolean data type calculated as follows: ${m}_{+} = \left\{\begin{matrix}1 & if m > 0 \\ 0 & otherwise\end{matrix}\right.$

In another form of this first configuration, the breathing parameter value *m*₊ may be a value representative of the percentage and/or ratio of time the fitted line is above or below zero, over the selected window or portion of discrete data points (e.g. 20 data points in the above example). For example, the breathing parameter value *m*₊ may be a Categorical value or variable or Categorical data-type calculated as follows: ${m}_{+} = \left\{\begin{matrix}1 & if m > \left|s\right| \\ \frac{1}{2} + \frac{m}{2 \left|s\right|} & if \left|m\right| \leq \left|s\right| \\ 0 & otherwise\end{matrix}\right.$

Where |*s*| is the absolute value of the slope of a fitted line and |*m*| is the absolute value of the mean value of the fitted line. In a second configuration, at step 805, the algorithm calculates a breathing parameter *m*₊ value based on or directly from the pre-processed flow parameter variation data *uₚₚ* representing the patient's breathing, and the function or line fitting of step 804 is omitted from the process. In this second configuration, the breathing parameter value *m*₊ is determined based on each incoming instantaneous pre-processed flow parameter variation data value *uₚₚ*. For example, the breathing parameter value *m*₊ may be a Boolean or binary value or variable or Boolean data type calculated as follows: ${m}_{+} = \left\{\begin{matrix}1 & if {u}_{pp} > 0 \\ 0 & otherwise\end{matrix}\right.$

The algorithm 800 may use any one of the above calculations or methods for calculating the updated breathing parameter value *m*₊ based on the incoming flow parameter variation data. In the above configurations, the breathing parameter *m*₊ may be considered to represent patient inspiration versus patient expiration.

In alternative configurations of the algorithm 800, it will be appreciated that other functions may be used to generate other suitable breathing parameter values based on the flow parameter variation data or based on the breathing parameter value *m*₊. For example, in one alternative configuration, a breathing parameter value may be generated that represents a rolling average of the patient inspiring. The rolling average breathing parameter value may be generated based on a cumulative inspiration value that is incremented by 1 each time the patient is determined to be inspiring according to the Boolean breathing parameter value *m*₊ (e.g. incrementing the cumulative inspiration value by 1 for each algorithm iteration in which *m₊* is 1, which occurs when the patient is inspiring). The rolling average breathing parameter value is determined each iteration by dividing the cumulative inspiration value by the cumulative number of algorithm iterations. In this example, the rolling average breathing parameter value is representative of the ratio of inspiration to total respiration.

As step 806, the algorithm calculates a breathing parameter ratio *Ti*/*Ttot* representing inspiration time relative to total respiration time based on a rolling or running average of the breathing parameter ratio *m₊.* In one configuration, the algorithm computes a running average of the breathing parameter *m*₊ using an exponential filter over a time period. In this example, the filter time period may be 45 seconds, but it could be any other suitable time period for example 1 minute or 2 minutes of flow data. Alternatively, any filter with low pass properties that averages over at least one breath period can be used. The output of the filter is the averaged breathing parameter *m+,* which represents the *Ti*/*Ttot* ratio. As explained further below, this breathing parameter ratio may be fed or output to other controller functions or processes related to monitoring, displaying data, alarms, notifications, and/or control of operating or therapy settings, such as steps 808 and/or 809.

In some configurations, step 806 of the algorithm may optionally comprise one or more additional processes. For example, in some configurations, the process of generating the breathing parameter ratio *Ti*/*Ttot* may optionally comprise one or more additional processing stages such as, but not limited to, noise correction processing and/or noise cleaning processes and/or noise filtering, and/or signal or data quality determination stages. Examples of these optional processes are provided in the following further alternative configuration of step 806.

In this alternative configuration of step 806, the algorithm can further process the breathing parameter value *m*₊ by performing noise correction using a noise function and a filter function. For example: $\frac{{T}_{i}}{{T}_{Tot}} , raw = f \left(σ,filt\left({m}_{+}\right)\right)$

Where *σ* is a signal to noise function with the property of being zero when the signal to noise ratio (SNR) is zero (i.e., entirely noise, no information signal present) and one when SNR is infinite or approaching infinity (i.e., zero noise). In this example, the function *σ* may be the same as the function described in PCT Application Publication No. WO 2020/178746, filed 4 March 2020. By way of example, *σ* may be represented by the following: $σ ≅ \frac{{SNR}^{2}}{1 + {SNR}^{2}}$

The function *filt()* can be any suitable function as described above previously in relation to step 806 such as, but not limited to, an exponential filter for example.

Once the $\frac{{T}_{i}}{{T}_{Tot}}$, raw value is obtained, it can be passed through yet another noise filter for cleaning: $\frac{{T}_{i}}{{T}_{Tot}} = filt \left(\frac{{T}_{i}}{{T}_{Tot}}, raw\right)$

Thus, a final suitable value for breathing parameter ratio $\frac{{T}_{i}}{{T}_{Tot}}$ can be obtained.

In order to assess the quality of the obtained breathing parameter ratio value, a variance number can be estimated. A standard deviation may be estimated using the variance number estimate.

In a configuration, the obtained breathing parameter ratio $\frac{{T}_{i}}{{T}_{Tot}}$ value can be deemed good, acceptable, or unacceptable (bad) depending on the magnitude of the estimated variance or standard deviation. For example, the acceptability criteria may be defined according to the following piecewise function: $Signal Quality = \left\{\begin{matrix}Bad , & 0.1 < std \left(\frac{{T}_{i}}{{T}_{Tot}}\right) \\ Acceptable , & 0.05 < std \left(\frac{{T}_{i}}{{T}_{Tot}}\right) < 0.1 \\ Good , & std \left(\frac{{T}_{i}}{{T}_{Tot}}\right) < 0.05\end{matrix}\right.$

Additionally, in one configuration, if $\frac{{T}_{i}}{{T}_{Tot}}$ (not the variance or standard variation thereof) is greater than 0.5 or less than 0.1, the signal quality can also be deemed unacceptable. For example, in some embodiments these values are not physically possible in the system and must therefore arise due to significant noise or error.

In this configuration, the output of step 806 is again a breathing parameter ratio representing *Ti*/*Ttot,* and optionally a signal quality indicator. Additionally, or alternatively, the algorithm may be configured to discard breathing parameter ratio data that is of unacceptable quality based on its associated signal quality indicator, i.e. the bad or low quality breathing parameter ratio data may discarded and/or not passed on to further processing stages of the algorithm. In one configuration, bad data generated in step 806 is not further processed in the algorithm by steps 808 and/or 809. In one example configuration, only breathing parameter ratio data having 'acceptable' or 'good' quality indicators, or alternatively only data having a 'good' quality indicator, may be passed on for output and/or further processing in the remaining stages of the algorithm 800.

### 2.5 Determining respiratory rate from pre-processed flow rate signal or flow rate data

In this example configuration of the algorithm 800, at step 807 the algorithm optionally receives or retrieves respiratory rate data representing or indicative of the current respiratory rate of the patient using the respiratory apparatus.

In one configuration, the respiratory rate data may be received from a respiratory rate detection device, monitor, or sensor associated or operatively connected to the respiratory apparatus.

In another configuration, the respiratory rate data may be calculated by another process or function operating in the controller based on other data or inputs from relevant sensors or devices of or operatively connected to the respiratory apparatus.

In another configuration, the respiratory rate data may be calculated by the algorithm from the new flow parameter data or flow parameter variation data from stages 801 and/or 803 respectively. By way of example, a value representing or indicative of respiratory rate may be generated by applying frequency domain analysis to the flow parameter data or flow parameter variation data. For example, the algorithm may implement techniques such as those disclosed in PCT Application Publication No. WO2019/102384, filed 22 November 2018. In one example configuration, the algorithm at step 807 may apply frequency analysis to the flow parameter or flow parameter variation data to determine or identify the dominant frequency in the data, and generate a respiration rate value based on the identified dominant frequency. In one configuration, the respiration rate value may be in the form of breaths per minute (BPM) or any other suitable metric.

### 2.6 Determining additional breathing parameters from primary breathing parameter ratio and respiratory rate

Optionally, at step 808, the algorithm 800 can be configured or is operable to generate one or more additional breathing parameters or ratios based on the primary breathing parameter ratio *Ti*/*Ttot* generated or output from step 806 and/or the respiratory rate data from 807. By way of example, such breathing parameters and/or ratios may include any one or more of: inspiration time *Ti,* expiration time *Te,* total respiration time *Ttot (Ti*+*Te),* ratio of inspiration time to expiration time *Ti*/*Te,* ratio of expiration time to inspiration time *Te*/*Ti,* ratio of expiration time to total respiration time *Te*/*Ttot.* Examples of how these parameters and/or ratios may be derived are outlined in the following equations: $Ttot = \frac{60}{RR} ,$ *where RR is provided in breaths per minute* $Ti = \left(Ti/Ttot\right) \times Ttot$ $Te / Ttot = 1 - \left(Ti/Ttot\right)$ $Te = \left(Te/Ttot\right) \times Ttot$ $\frac{Ti}{Te} = \left(Ti/Ttot\right) / \left(Te/Ttot\right)$ $\frac{Te}{Ti} = \left(Te/Ttot\right) / \left(Ti/Ttot\right)$

### 2. 7 Applications, actions and/or functions based on determined breathing parameter data

At step 809, the algorithm may execute one or more actions and/or functions based on any one or more of the calculated or updated breathing parameter data and/or ratios. In some configurations, the algorithm 800 may output the primary breathing parameter ratio *(Ti*/*Ttot)* from step 806, which is then monitored, analysed, displayed and/or used to trigger alarms, notifications, or to modify operational or therapy settings (e.g. flow rate setting or other settings) of the respiratory apparatus. In other configurations, the algorithm may output one or more of the additional breathing parameter values or ratios calculated at step 808, for similar such functions, e.g. monitoring, analysing, displaying, alarms, notifications, controlling operating and/or therapy settings.

Various example applications and uses of the calculated breathing parameters and/or ratios are explained below, by way of non-limiting example only. Some potential benefits and/or advantages of some configurations and/or embodiments are also explained.

### First example application

In this example application, estimates for the breathing parameters may be a valuable indicator of therapy efficacy, prompting a user or health professional to adjust therapy parameters such as device flow rate or the type of patient interface if a patient is not responding positively to the current therapy parameters. Prompts to adjust therapy parameters may be presented visually on a device display.

### Second example application

In this example application, the breathing parameter determination algorithm may be able to provide the respiratory apparatus controller with a greater understanding of the patients' breathing behaviour and therapy efficacy during a therapy session. This may be provided by the algorithm estimating algorithmically new breathing parameters and/or ratios including and based on inspiratory time, expiratory time, and/or total respiration time. The breathing parameters generated by the algorithm may be used to visually present trend data (for example, one or more time series plots of one or more of the breathing parameters and/or ratios) and in some configurations and/or embodiments to provide alarms, prompts or notifications to a user or clinician based on certain thresholds. The breathing parameter determination algorithm as described is advantageous because the apparatus provides additional parameters that are indicative of patient respiratory health or patient respiratory condition. For example, if the inspiratory time to total respiratory time can be indicative of a patient in respiratory distress or the state of their respiratory health. The determined breathing parameters (i.e. the specific ratios) as described can be indicative of if high flow therapy is being effective in a patient. The trend of the determined breathing parameters can be indicative of an improvement of a patient's respiratory health or a worsening of the patient's respiratory health.

In some configurations and/or embodiments, the respiratory apparatus controller may automatically adjust therapy parameters (e.g., flow rate) depending on whether the breathing parameters and/or ratios exceed certain thresholds. For example, in such configurations and/or embodiments, the respiratory apparatus controller may automatically increase the flow rate of the apparatus output if the $\frac{{T}_{i}}{{T}_{e}}$ ratio is below a certain threshold configured (i.e. set) by a clinician. For example, the clinician may set via the device display a set ratio of inspiratory time to total respiratory time, or inspiratory time to expiratory time. The respiratory apparatus controller is configured to determine the ratio and compare with the set ratio, and based on the difference control the flow generator to increase flow or decrease flow automatically to achieve a set ratio.

### Third example application

In this example application, in the context of nasal high flow therapy (NHF), the algorithm may provide for accurate estimation of inspiratory *Ti* and expiratory *Te* times/periods and their ratio.

For a healthy adult patient, *Tₑ* should be approximately equal to 3*Tᵢ* (i.e. *Tₑ* ≈ 3*Tᵢ* or $\frac{{T}_{e}}{{T}_{i}} \approx 3$ or conventionally $\frac{{T}_{i}}{{T}_{e}} \approx 0.33$). As health worsens, breathing effort increases and the value of *Tₑ* tends toward the value of *Tᵢ* (i.e. *Tₑ* → *Tᵢ* or $\frac{{T}_{i}}{{T}_{e}} \to 1$).

It is known that effective NHF therapy will increase patient *Tₑ,* increasing dead space wash-out and allowing the patient to expire more gas, thereby increasing their tidal volume. If *Tᵢ* is approximately constant and *Tₑ* is increasing/increases during NHF, the ratio $\frac{{T}_{i}}{{T}_{e}}$ will decrease. This would be indicative of therapy being effective. Conversely, an increasing $\frac{{T}_{i}}{{T}_{e}}$ ratio would be indicative of the patient condition worsening or ineffective therapy - in either case, therapy adjustment may be required. The $\frac{{T}_{i}}{{T}_{e}}$ ratio may also be monitored for trends toward lower and/or upper thresholds, as a ratio that is either too high or too low may indicate ineffective therapy and/or an adverse patient condition.

Because *Tᵢ, Tₑ, Tₜₒₜ,* $\frac{{T}_{i}}{{T}_{e}} , \frac{{T}_{e}}{{T}_{i}} , \frac{{T}_{i}}{{T}_{tot}} , \frac{{T}_{e}}{{T}_{tot}}$ are metrics of physiological parameters, while they are accurate, they may tend to fluctuate and are also highly subjective, patient-to-patient. Therefore, in some configurations, triggering of alarms or notifications may be based more on trend-analysis of the parameters, rather than comparing the parameters directly to thresholds. For example, a time-based trend in any of these parameters could be displayed in a visual format (e.g. a plot, chart or graph) for a clinician to monitor, and/or alarms or notifications could be automatically triggered based on trend-analysis thresholds, e.g. prolonged significant upward or downward trends for example.

In some configurations and/or embodiments, the respiratory apparatus controller may automatically adjust therapy parameters (e.g., flow rate) depending on whether the breathing parameter ratios and/or their trends exceed certain thresholds. The controller may automatically control flow rate based on the relationship of the breathing parameter ratios and/or their trends relative to clinician set thresholds. For example, in such configurations and/or embodiments, the respiratory apparatus controller may automatically increase the flow rate of the apparatus output if there is a consistent downward trend of $\frac{{T}_{i}}{{T}_{e}}$ (that is, $\frac{{T}_{i}}{{T}_{e}}$ is tending towards 1). The trend threshold may be configured by a clinician.

### Fourth example application

In this example application, the algorithm may provide a method of determining and monitoring breathing parameter data (e.g. breathing times of *Ti, Te, Ttot)* and their various ratios to allow for improving therapy control. The algorithm computations to determine *Tᵢ* and *Tₑ* are less influenced by the unavoidable sources of error present when using operating data from a typical NHF therapy session.

The algorithm provides additional breathing parameters to the controller and facilitates additional means of adjusting patient therapy, in the context of NHF where estimating breathing parameters is very difficult due to the unsealed patient interface.

Estimating the $\frac{{T}_{i}}{{T}_{e}}$ ratio may provide a robust approach to determining an indicator of NHF therapy efficacy and the patient's breathing performance. Compared to monitoring breathing parameters such as tidal volume, or even minute ventilation and peak inspiratory flow, the $\frac{{T}_{i}}{{T}_{e}}$ ratio suffers much less from the sources of error inherent with using an unsealed nasal cannula.

A major issue which is known is insufficient flow rate being set by users or health professionals on NHF devices. The algorithm may alleviate this issue by providing feedback prompts to adjust flow rate, if the controller determines that therapy efficacy is insufficient for the patient's condition based on the breathing parameters determined by the algorithm.

In some applications, alarms/notifications/indicators may be configured to trigger based on certain thresholds related to the breathing parameters generated and/or computed by the algorithm. Users or health professionals will be provided with this additional information about patient health and NHF therapy efficacy during the therapy session, and this may encourage more accurate adjustment of therapy parameters, leading to improved patient outcomes.

### 3. Terminology

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to".

Features, materials, characteristics, or groups described in conjunction with a particular aspect, embodiment, or example are to be understood to be applicable to any other aspect, embodiment or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive.

Furthermore, certain features that are described in this disclosure in the context of separate implementations can also be implemented in combination in a single implementation. Conversely, various features that are described in the context of a single implementation can also be implemented in multiple implementations separately or in any suitable subcombination.

Moreover, while operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some embodiments, the actual steps taken in the processes illustrated and/or disclosed may differ from those shown in the figures. Depending on the embodiment, certain of the steps described above may be removed, others may be added. Furthermore, the features and attributes of the specific embodiments disclosed above may be combined in different ways to form additional embodiments, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments or that one or more embodiments necessarily include logic for deciding, with or without user input or prompting, whether these features, elements, and/or steps are included or are to be performed in any particular embodiment. Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount.

## Claims

1. A respiratory apparatus (10) that is configured to provide a flow of gases to a user for respiratory therapy comprising:
a flow generator (50B) that is operable to generate a flow of gases;
a controller (19) that is operatively connected to the flow generator and operable to control a flow rate of the flow of gases by controlling the flow generator, wherein the controller is configured during operation to:
receive flow rate data (701) indicative of or representing the flow rate of the flow of gases;
process the flow rate data to extract or generate breathing data (702) indicative of or representing the patient's breathing or respiration from the flow rate data; and
process the breathing data to calculate one or more breathing parameter ratios (703) representative of the ratio between inspiration time and/or expiration time to total respiration time for the patient's breathing cycle by:
fitting a function or line to a selected portion of the breathing data,
calculating a breathing parameter value based at least partly on one or more parameters defining the fitted function or line, and
determining one or more of the breathing parameter ratios based on a rolling average of the breathing parameter value, and
wherein the respiratory apparatus is configured or operable to deliver high flow therapy to a patient via an unsealed interface (51).

2. A respiratory apparatus according to claim 1 wherein the breathing parameter value is a Boolean or Categorical value or data type that is calculated or determined based on one or more parameters defining the fitted function or line.

3. A respiratory apparatus according to claim 1 or claim 2 wherein the controller (19) is further configured to:
receive or calculate respiratory rate data (704) representing or indicative of the patient's respiratory rate, and
calculate one or more additional breathing parameter ratios and/or breathing parameters (705) based on the initially calculated breathing parameter ratio(s) and the respiratory rate data, and wherein the one or more additional breathing parameter ratios calculated comprise any additional ratio between any combination or permutation of two of the following breathing parameters: inspiration time, expiration time, and total respiration time.

4. A respiratory apparatus according to claim 3 wherein the one or more additional breathing parameters calculated comprise any one or more of the following: inspiration time, expiration time, and/or total respiration time.

5. A respiratory apparatus according to claim 3 or claim 4 further comprising a device or sensor operatively connected to the apparatus, and wherein the respiratory rate data is received or retrieved from the device or sensor; and/or wherein the controller (19) is configured to calculate the respiratory rate data from the breathing data extracted from the flow rate data.

6. A respiratory apparatus according to any one of claims 1-5 wherein the controller (19) is configured to display one or more of the calculated breathing parameter ratios and/or breathing parameters on a display (630) of the apparatus (10), and wherein the controller (10) is configured to display one or more of the calculated breathing parameter ratios and/or breathing parameters numerically and/or as a graph, plot or chart.

7. A respiratory apparatus according to any one of claims 1-6 wherein the controller (19) is configured to trigger alarms and/or notifications for display (630) on the respiratory apparatus (10) based on analysing the calculated breathing parameter ratios and/or breathing parameters, and wherein the controller is configured to apply trend analysis to one or more of the calculated breathing parameter ratios and/or breathing parameters, and triggers alarms and/or notifications for display based on the trend analysis and configurable trend thresholds.

8. A respiratory apparatus according to any one of claims 1-7 wherein the controller (19) is configured to modify or alter operational and/or therapy settings based on the calculated breathing parameter ratios and/or breathing parameters.

9. A respiratory apparatus according to any one of claims 1-8 wherein the respiratory apparatus (10) further comprises a humidifier (52) that is configured to heat and/or humidify the flow of gases, and wherein the flow generator (50B) and humidifier are integrated within or provided in a common main housing (100).

10. A respiratory apparatus according to claim 9 further comprising one or more flow rate sensors in the main housing (100) that are configured to sense the flow rate of the flow of gases in a flow path of the main housing, and wherein the flow rate data (902) is received from the one or more flow rate sensors.

## Patentansprüche

1. Beatmungseinrichtung (10), die konfiguriert ist, um einem Benutzer eine Strömung von Gasen für eine Beatmungstherapie bereitzustellen, umfassend:
einen Strömungserzeuger (50B), der betriebsfähig ist, um eine Strömung von Gasen zu erzeugen;
eine Steuerung (19), die mit dem Strömungserzeuger wirkverbunden ist und betriebsfähig ist, um eine Strömungsgeschwindigkeit der Strömung von Gasen durch Steuern des Strömungserzeugers zu steuern, wobei die Steuerung während eines Betriebs konfiguriert ist zum:
Empfangen von Strömungsgeschwindigkeitsdaten (701), die die Strömungsgeschwindigkeit der Strömung von Gasen angeben oder darstellen;
Verarbeiten der Strömungsgeschwindigkeitsdaten, um Atemdaten (702), die das Atmen oder die Respiration eines Patienten angeben oder darstellen, aus den Strömungsgeschwindigkeitsdaten zu extrahieren oder zu erzeugen; und
Verarbeiten der Atemdaten, um einen oder mehrere Atemparameterverhältnisse (703) zu berechnen, die darstellend für das Verhältnis zwischen Inspirationszeit und/oder Exspirationszeit zu einer Gesamtatemzeit für den Atemzyklus des Patienten sind, durch:
Anpassen einer Funktion oder Linie an einen ausgewählten Abschnitt der Atemdaten,
Berechnen eines Atemparameterwerts basierend mindestens teilweise auf einem oder mehreren Parametern, die die angepasste Funktion oder Linie definieren, und
Bestimmen eines oder mehrerer der Atemparameterverhältnisse basierend auf einem gleitenden Mittelwert des Atemparameterwerts, und
wobei die Beatmungseinrichtung konfiguriert oder betriebsfähig ist, um einem Patienten über eine nicht abgedichtete Schnittstelle (51) eine High-Flow-Therapie zuzuführen.

2. Beatmungseinrichtung nach Anspruch 1, wobei der Atemparameterwert ein boolescher oder kategorialer Wert oder Datentyp ist, der basierend auf einem oder mehreren Parametern berechnet oder bestimmt wird, die die angepasste Funktion oder Linie definieren.

3. Beatmungseinrichtung nach Anspruch 1 oder 2, wobei die Steuerung (19) ferner konfiguriert ist zum:
Empfangen oder Berechnen von Atemfrequenzdaten (704), die die Atemfrequenz des Patienten darstellen oder angeben, und
Berechnen eines oder mehrerer zusätzlicher Atemparameterverhältnisse und/oder Atemparameter (705) basierend auf dem oder den anfänglich berechneten Atemparameterverhältnissen und den Atemfrequenzdaten, und wobei das eine oder die mehreren berechneten zusätzlichen Atemparameterverhältnisse ein beliebiges zusätzliches Verhältnis zwischen einer beliebigen Kombination oder Permutation von zwei der folgenden Atemparameter umfassen: Inspirationszeit, Exspirationszeit und Gesamtatemzeit.

4. Beatmungseinrichtung nach Anspruch 3, wobei der eine oder die mehreren berechneten zusätzlichen Atemparameter einen oder mehrere der Folgenden umfassen: Inspirationszeit, Exspirationszeit und/oder Gesamtatemzeit.

5. Beatmungseinrichtung nach Anspruch 3 oder 4, ferner umfassend eine Einrichtung oder einen Sensor, die oder der mit der Einrichtung wirkverbunden ist, und wobei die Atemfrequenzdaten von der Einrichtung oder dem Sensor empfangen oder abgerufen werden; und/oder wobei die Steuerung (19) konfiguriert ist, um die Atemfrequenzdaten aus den Atemdaten zu berechnen, die aus den Strömungsgeschwindigkeitsdaten extrahiert werden.

6. Beatmungseinrichtung nach einem der Ansprüche 1 bis 5, wobei die Steuerung (19) konfiguriert ist, um eines oder mehrere der berechneten Atemparameterverhältnisse und/oder Atemparameter auf einer Anzeige (630) der Einrichtung (10) anzuzeigen, und wobei die Steuerung (10) konfiguriert ist, um eines oder mehrere der berechneten Atemparameterverhältnisse und/oder Atemparameter numerisch und/oder als einen Graphen, eine Kurve oder ein Diagramm anzuzeigen.

7. Beatmungseinrichtung nach einem der Ansprüche 1 bis 6, wobei die Steuerung (19) konfiguriert ist, um Alarme und/oder Benachrichtigungen für die Anzeige (630) auf der Beatmungseinrichtung (10) basierend auf dem Analysieren der berechneten Atemparameterverhältnisse und/oder Atemparameter auszulösen, und wobei die Steuerung konfiguriert ist, um eine Trendanalyse auf eines oder mehrere der berechneten Atemparameterverhältnisse und/oder Atemparameter anzuwenden, und Alarme und/oder Benachrichtigungen für die Anzeige basierend auf der Trendanalyse und konfigurierbaren Trendschwellenwerten auslöst.

8. Beatmungseinrichtung nach einem der Ansprüche 1 bis 7, wobei die Steuerung (19) konfiguriert ist, um Betriebs- und/oder Therapieeinstellungen basierend auf den berechneten Atemparameterverhältnissen und/oder Atemparametern zu modifizieren oder zu ändern.

9. Beatmungseinrichtung nach einem der Ansprüche 1 bis 8, wobei die Beatmungseinrichtung (10) ferner einen Befeuchter (52) umfasst, der konfiguriert ist, um die Strömung von Gasen zu erwärmen und/oder zu befeuchten, und wobei der Strömungsgenerator (50B) und der Befeuchter innerhalb eines gemeinsamen Hauptgehäuses (100) integriert oder bereitgestellt sind.

10. Beatmungseinrichtung nach Anspruch 9, ferner umfassend einen oder mehrere Strömungsgeschwindigkeitssensoren in dem Hauptgehäuse (100), die konfiguriert sind, um die Strömungsgeschwindigkeit der Strömung von Gasen in einem Strömungsweg des Hauptgehäuses zu erfassen, und wobei die Strömungsgeschwindigkeitsdaten (902) von dem einen oder den mehreren Strömungsgeschwindigkeitssensoren empfangen werden.

## Revendications

1. Appareil respiratoire (10) qui est conçu pour fournir un écoulement de gaz à un utilisateur pour une thérapie respiratoire comprenant :
un générateur d'écoulement (50B) qui est fonctionnel pour générer un écoulement de gaz ;
une unité de commande (19) qui est fonctionnellement connectée au générateur d'écoulement et fonctionnelle pour commander un débit d'écoulement de l'écoulement de gaz en commandant le générateur d'écoulement, dans lequel l'unité de commande est configurée pendant le fonctionnement pour :
recevoir des données de débit d'écoulement (701) indiquant ou représentant le débit d'écoulement de l'écoulement de gaz ;
traiter les données de débit d'écoulement pour extraire ou générer des données ventilatoires (702) indiquant ou représentant la ventilation ou la respiration du patient à partir des données de débit d'écoulement ; et
traiter les données ventilatoires pour calculer un ou plusieurs rapports de paramètres ventilatoires (703) représentatifs du rapport entre le temps d'inspiration et/ou le temps d'expiration et le temps total de respiration pour le cycle ventilatoire du patient par :
l'ajustement d'une fonction ou d'une ligne à une partie sélectionnée des données ventilatoires,
le calcul d'une valeur de paramètre de ventilation en fonction au moins partiellement d'un ou plusieurs paramètres définissant la fonction ou ligne ajustée, et
la détermination d'un ou plusieurs des rapports de paramètres ventilatoires en fonction d'une moyenne mobile de la valeur de paramètre de ventilation, et
dans lequel l'appareil respiratoire est configuré ou fonctionnel pour délivrer une thérapie à haut débit à un patient par l'intermédiaire d'une interface non étanche (51).

2. Appareil respiratoire selon la revendication 1 dans lequel la valeur de paramètre de ventilation est une valeur ou un type de données booléen(ne) ou catégoriel(le) qui est calculé ou déterminé en fonction d'un ou plusieurs paramètres définissant la fonction ou ligne ajustée.

3. Appareil respiratoire selon la revendication 1 ou la revendication 2 dans lequel l'unité de commande (19) est configurée en outre pour :
recevoir ou calculer des données de fréquence respiratoire (704) représentant ou indiquant la fréquence respiratoire du patient, et
calculer un ou plusieurs rapports de paramètres ventilatoires et/ou paramètres ventilatoires (705) supplémentaires en fonction du ou des rapport(s) de paramètres ventilatoires initialement calculé(s) et des données de fréquence respiratoire, et dans lequel le ou les rapports de paramètres ventilatoires supplémentaires calculés comprennent l'un quelconque rapport supplémentaire entre l'une quelconque combinaison ou permutation de deux des paramètres ventilatoires suivants : temps d'inspiration, temps d'expiration et temps total de respiration.

4. Appareil respiratoire selon la revendication 3 dans lequel le ou les paramètres ventilatoires supplémentaires calculés comprennent l'un quelconque ou plusieurs quelconques des suivants : temps d'inspiration, temps d'expiration et/ou temps total de respiration.

5. Appareil respiratoire selon la revendication 3 ou la revendication 4 comprenant en outre un dispositif ou capteur connecté fonctionnellement à l'appareil, et dans lequel les données de fréquence respiratoire sont reçues ou récupérées du dispositif ou du capteur ; et/ou dans lequel l'unité de commande (19) est configurée pour calculer les données de fréquence respiratoire à partir des données ventilatoires extraites des données de débit d'écoulement.

6. Appareil respiratoire selon l'une quelconque des revendications 1 à 5 dans lequel l'unité de commande (19) est configurée pour afficher un ou plusieurs des rapports de paramètres ventilatoires et/ou paramètres ventilatoires calculés sur un affichage (630) de l'appareil (10), et dans lequel l'unité de commande (10) est configurée pour afficher un ou plusieurs des rapports de paramètres ventilatoires et/ou paramètres ventilatoires calculés numériquement et/ou en tant que graphe, tracé ou graphique.

7. Appareil respiratoire selon l'une quelconque des revendications 1 à 6 dans lequel l'unité de commande (19) est configurée pour déclencher des alarmes et/ou des notifications pour affichage (630) sur l'appareil respiratoire (10) en fonction d'une analyse des rapports de paramètres ventilatoires et/ou paramètres ventilatoires calculés, et dans lequel l'unité de commande est configurée pour appliquer une analyse de tendance à un ou plusieurs des rapports de paramètres ventilatoires et/ou paramètres ventilatoires calculés, et déclenche des alarmes et/ou notifications pour affichage en fonction de l'analyse de tendance et de seuils de tendance configurables.

8. Appareil respiratoire selon l'une quelconque des revendications 1 à 7 dans lequel l'unité de commande (19) est configurée pour modifier ou adapter des réglages fonctionnels et/ou thérapeutiques en fonction des rapports de paramètres ventilatoires et/ou paramètres ventilatoires calculés.

9. Appareil respiratoire selon l'une quelconque des revendications 1 à 8 dans lequel l'appareil respiratoire (10) comprend en outre un humidificateur (52) qui est configuré pour chauffer et/ou humidifier l'écoulement de gaz, et dans lequel le générateur d'écoulement (50B) et l'humidificateur sont intégrés au sein de, ou fournis dans, un logement principal commun (100).

10. Appareil respiratoire selon la revendication 9 comprenant en outre un ou plusieurs capteurs de débit d'écoulement dans le logement principal (100) qui sont configurés pour capter le débit d'écoulement de l'écoulement de gaz dans un chemin d'écoulement du logement principal, et dans lequel les données de débit d'écoulement (902) sont reçues du ou des capteurs de débit d'écoulement.
